# EUROPEAN PATENT APPLICATION

(11) **EP 4 154 856 A1**
(43) Date of publication of application: **29.03.2023**
(21) Application number: 21808272.5
(22) Date of filing: 19.05.2021
(51) Int. Cl.: A61F 13/532, A61F 13/49, A61F 13/535

(54) **DISPOSABLE DIAPER**

(30) Priority: 20.05.2020 WO PCT/JP2020/020021
(71) Applicant: Kao Corporation, Chuo-ku Tokyo 103-8210 (JP)
(72) Inventor: TSUGE, Kyoko, Haga-gun, Tochigi 321-3497 (JP); FUKUDA, Yuko, Haga-gun, Tochigi 321-3497 (JP); OKUDA, Yasuyuki, Haga-gun, Tochigi 321-3497 (JP)
(74) Representative: Vossius & Partner Patentanwälte Rechtsanwälte mbB
(86) International application number: PCT/JP2021/019074
(87) International publication number: WO 2021/235504

(57) **Abstract**

An absorbent member (4) of a diaper 1 includes, in a crotch portion (C): a central absorbent member (4C) extending in the longitudinal direction; a pair of side absorbent members (4S, 4S) respectively located on both sides, in the width direction, of the central absorbent member, the side absorbent members configured to stand up from a skin-facing surface side of the central absorbent member in a worn state; and a pair of bending-guide portions (45, 45). An absorbent core (40) of the absorbent member (4) has: a central region (41) in the central absorbent member (4C); and side regions (43) in the respective side absorbent members (4S), the central region (41) and the side regions (43) being divided by the pair of bending-guide portions. The bending-guide portion (45) is constituted by a slit or a groove, and the width thereof before absorbing liquid is from 10 to 30 mm. In the absorbent core (41) in a reference swollen state, the width of the side region (43) is greater than the width of the central region (41), and half the width of the central region (41) is 0.7 times or less of the thickness of each side region (43).

## Description

### Technical Field

The present invention relates to a disposable diaper.

### Background Art

A typical absorbent article, such as a disposable diaper, is provided with an absorbent member including an absorbent core, as a liquid-absorbing section for absorbing body fluid such as urine. From the viewpoint of improving leakage preventability and fittability to the wearer's skin, there have been proposed absorbent articles including an absorbent core having a plurality of regions. The plurality of regions are divided by depressions, such as slits or grooves, or by varying the thickness, basis weight, etc., of the respective regions.

### Citation List

### Patent Literature

Patent Literature 1: JP 2006-247363A
Patent Literature 2: WO2014/200794 A1

### Summary of Invention

The present invention relates to a disposable diaper including a topsheet, a backsheet, and an absorbent member arranged between the topsheet and the backsheet, the absorbent member including an absorbent core, the disposable diaper having a longitudinal direction and a width direction orthogonal to the longitudinal direction, the disposable diaper having a rear portion to be arranged on a wearer's rear side when worn, a front portion to be arranged on the wearer's front side when worn, and a crotch portion located between the front portion and the rear portion.

Preferably, in the crotch portion, the absorbent member includes: a central absorbent member extending in the longitudinal direction; a pair of side absorbent members respectively located on both sides, in the width direction, of the central absorbent member, the side absorbent members configured to stand up from a skin-facing surface side of the central absorbent member in a worn state; and a pair of bending-guide portions, each of the bending-guide portions being formed between the central absorbent member and the respective side absorbent member.

Preferably, the absorbent core has a central region in the central absorbent member and side regions in the respective side absorbent members, the central region and the side regions being divided by the pair of bending-guide portions.

Preferably, the bending-guide portion is constituted by a slit or a groove.

Preferably, a width of the bending-guide portion before absorbing liquid is from 10 to 30 mm.

Preferably, in a reference swollen state achieved by injecting 160 g of artificial urine to an injection point located 7 cm away toward the front portion side in the longitudinal direction from a center position of the disposable diaper, which is at a center in the longitudinal direction and at a center in the width direction, and leaving the disposable diaper to stand for 5 minutes, a width of the side region of the absorbent core is greater than a width of the central region.

Preferably, in the reference swollen state, half the width of the central region is 0.7 times or less of a thickness of each side region.

### Brief Description of Drawings

[Fig. 1] Fig. 1 is a perspective view illustrating an embodiment of a disposable diaper of the present invention.
[Fig. 2] Fig. 2 is a developed plan view schematically illustrating a skin-facing surface side of the disposable diaper illustrated in Fig. 1 in a spread-open and stretched state.
[Fig. 3] Fig. 3 is a cross-sectional view taken along line II-II illustrated in Fig. 2.
[Fig. 4] Fig. 4 is a cross-sectional view taken along the width direction of an absorbent member, illustrating a central region and side regions of the absorbent member in a reference swollen state.
[Fig. 5] Fig. 5 is a cross-sectional view of the diaper in a swollen state taken along the diaper's width direction, illustrating, together with the wearer's crotch region, the diaper's crotch portion having been deformed in a worn state.
[Fig. 6] Fig. 6 is an explanatory diagram for illustrating a method for measuring 30-mm compression load.
[Fig. 7] Fig. 7 is a cross-sectional view illustrating the thickness of the absorbent member when the absorbent member in a reference swollen state has been folded along the longitudinal direction.
[Fig. 8] Fig. 8 is a plan view illustrating a positional relationship between the absorbent core illustrated in Fig. 2 and leak-proof cuffs.
[Fig. 9] Fig. 9 is a cross-sectional view of the absorbent member illustrated in Fig. 3.
[Fig. 10] Fig. 10 is a plan view illustrating another embodiment of an absorbent core according to the present invention.
[Fig. 11] Fig. 11 is a diagram corresponding to Fig. 10, illustrating yet another embodiment of an absorbent core according to the present invention.
[Fig. 12] Figs. 12(a) and 12(b) are diagrams corresponding to Fig. 4, illustrating other embodiments of absorbent cores according to the present invention.
[Fig. 13] Fig. 13 is a graph showing the results of the frontal plane hip joint angle in Evaluation I of gait influence degree.
[Fig. 14] Fig. 14 is a graph showing the results of step width (on force plate) in Evaluation I of gait influence degree.
[Fig. 15] Fig. 15 is a graph showing the results of the total movement distance of the body's barycenter in the left-right direction in Evaluation I of gait influence degree.
[Fig. 16] Fig. 16 is a graph showing the results of step width (on force plate) in Evaluation II of gait influence degree.
[Fig. 17] Fig. 17 is a graph showing the results of step width based on skeleton information in Evaluation II of gait influence degree.
[Fig. 18] Fig. 18 is a graph showing the results of knee-to-knee distance in Evaluation II of gait influence degree.

### Description of Embodiments

Applicant has previously proposed, as an absorbent article including an absorbent core having a plurality of regions, a disposable diaper provided with an absorbent member including a central absorbent member, a pair of side absorbent members, and gaps formed between these absorbent members, the disposable diaper being configured such that, in the crotch portion, both lateral sides of an absorbent assembly including an absorbent core are capable of standing up (Patent Literature 1).

Patent Literature 2 discloses an absorbent article including one or more area(s) substantially free of absorbent material, wherein a top side of a core wrap, which is for enclosing the absorbent material, is attached to a bottom side of the core wrap through the one or more area(s). When the absorbent material swells, the core wrap forms one or more channel(s) along the area(s).

Absorbent cores typically include absorbent material such as water-absorbent polymer, and hence swell upon absorption of body fluid such as urine. If the absorbent core swells excessively, the entire disposable diaper may become too bulky. This may cause the disposable diaper to abut against the wearer's femoral regions and restrict the wearer's movement. Particularly, if the movement around the femoral region is restricted, walking may be inhibited. This may become a hinderance when a toddler etc. is learning to walk or when an elderly person etc. is performing walking rehabilitation. Unfortunately, Patent Literatures 1 and 2 do not disclose techniques for facilitating movement around the wearer's femoral regions even when the absorbent core has swollen.

The present invention relates to a disposable diaper capable of facilitating the wearer's movement even when the absorbent core has swollen.

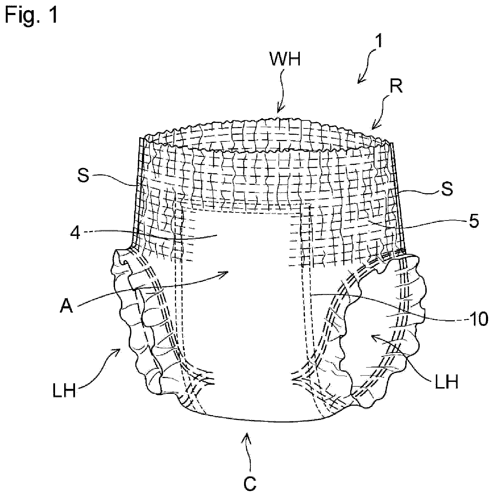

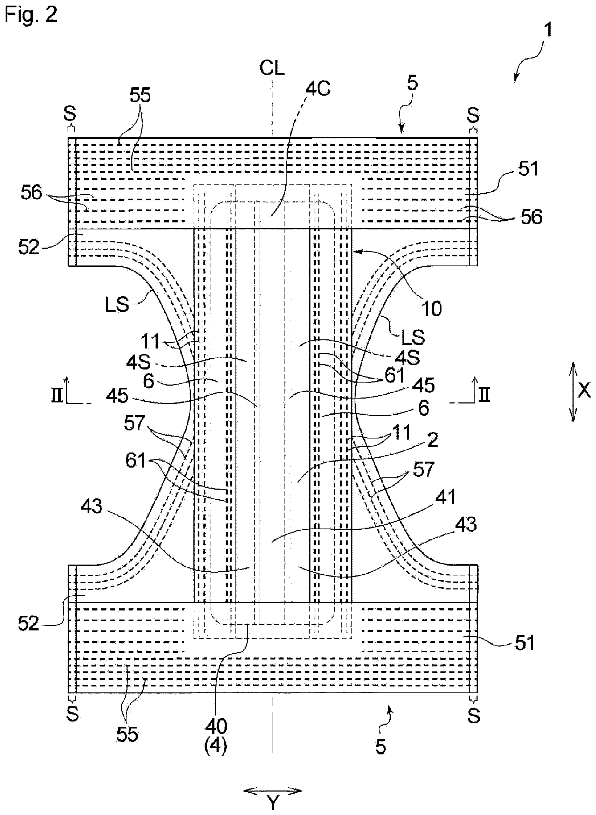

The present invention is described below according to preferred embodiments thereof with reference to the drawings. Figs. 1 and 2 illustrate a pull-on disposable diaper 1 (also referred to hereinafter simply as "diaper 1") which is an embodiment of a disposable diaper of the present invention. As illustrated in Fig. 1, the diaper 1 includes: a front portion A to be arranged on the wearer's front side in a state where the diaper 1 is worn; a rear portion B to be arranged on the wearer's rear side in a state where the diaper 1 is worn; and a crotch portion C located between the front portion and the rear portion. The diaper 1 has a longitudinal direction X corresponding to the wearer's front-rear direction-i.e., corresponding to a direction extending from the front portion A to the rear portion B via the crotch portion C-and a width direction Y orthogonal to the longitudinal direction. As illustrated in Fig. 2, the diaper 1 is formed so as to have left-right symmetry with respect to a longitudinal center line CL that extends along the longitudinal direction X and that divides the diaper 1 equally in two in the width direction Y.

The diaper 1 includes, in a central portion in the width direction Y, an absorbent assembly 10 including an absorbent member 4, and the diaper also includes an outer cover 5 provided on the non-skin-facing surface side of the absorbent assembly 10-i.e., provided on a side farther from the wearer's body than the absorbent assembly 10.

The absorbent member 4 extends between the front portion A and the rear portion B, and includes: a liquid-retentive absorbent core 40; and a core-wrap sheet 48 that comes into contact with the outer surface of the absorbent core 40 and covers the same.

In the outer cover 5, a pair of side seals S, S, a waist opening WH through which the wearer's body passes, and a pair of leg openings LH (only one is illustrated in the figure) through which the wearer's legs pass are formed by joining together the outer cover 5's both lateral side edge portions, which extend along the longitudinal direction X, in the respective front portion A and the rear portion B by using a known joining means such as an adhesive, e.g. a hot-melt adhesive, heat sealing, ultrasonic sealing, etc.

In this Description, the "skin-facing surface" is the surface of the absorbent article such as a disposable diaper, as well as its constituent members (e.g., the topsheet), that faces the wearer's skin side when the absorbent article is worn-i.e., the side relatively closer to the wearer's skin-whereas the "non-skin-facing surface" is the surface of the absorbent article, as well as its constituent members, that faces the opposite side from the wearer's skin side when the absorbent article is worn-i.e., the side relatively farther from the wearer's skin. Herein, "when worn" and "worn state/wearing state" refer to a state in which the absorbent article is maintained in its ordinary, proper wearing/attachment position-i.e., the correct wearing/attachment position of the absorbent article.

The absorbent assembly 10 has a rectangular shape in a planar view in a spread-open and stretched state of the diaper 1, as illustrated in Fig. 2. The absorbent assembly 10 extends in the longitudinal direction X from the front portion A to the rear portion B, and is arranged in the outer cover 5's central portion in the width direction Y in a manner that the absorbent assembly's longitudinal direction matches the longitudinal direction X of the diaper 1 in its spread-open and stretched state, and is joined to the outer cover 5 by an adhesive.

Herein, a "spread-open and stretched state" of the diaper 1 refers to a state in which the side seals S of the diaper 1 are torn apart to bring the diaper in a spread-out (developed) state, and the elastic members in various parts of the spread-out diaper 1 are stretched so that the diaper is spread to its designed size (size when the diaper is spread out in a planar manner in a state where the effect of the elastic members is completely eliminated).

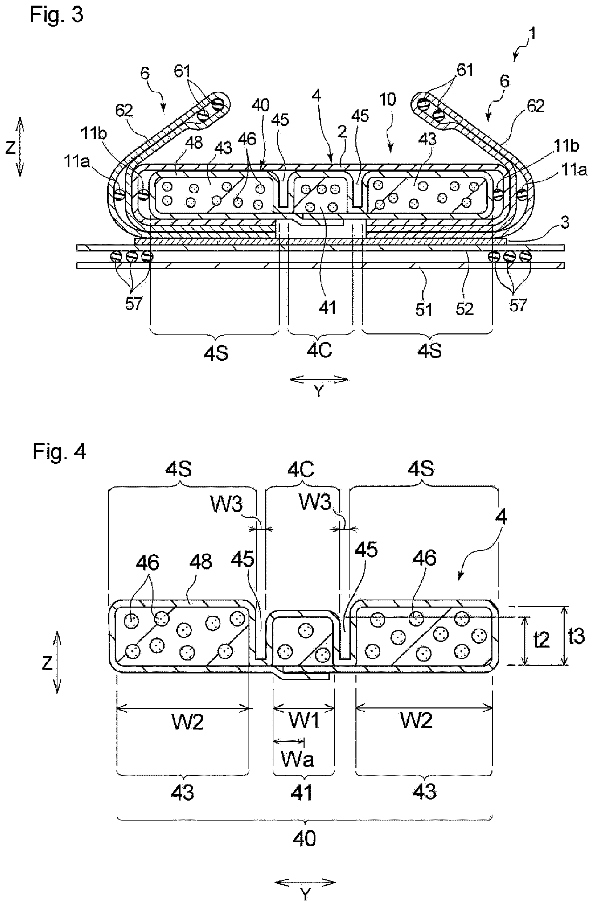

As illustrated in Fig. 3, the absorbent assembly 10 includes: a liquid-permeable topsheet 2 forming the skin-facing surface; a liquid-impermeable or sparingly liquid-permeable or water-repellent backsheet 3 forming the non-skin-facing surface; and a liquid-retentive absorbent member 4 interposed between the two sheets 21, 22. The two sheets and the absorbent member are integrated by a known joining means such as an adhesive.

In the present embodiment, the topsheet 2 covers the skin-facing surface side of the absorbent member 4, and the topsheet's both side end portions are folded back toward the non-skin-facing surface side of the absorbent member 4.

For the topsheet 2 and the backsheet 3, it is possible to use, without particular limitation, one of various materials conventionally used in this type of absorbent article. For example, for the topsheet 2, it is possible to use one of various nonwoven fabrics or a porous film. For the backsheet 3, for example, it is possible to use a resin film or a laminate of a resin film and a nonwoven fabric.

In a planar view as illustrated in Fig. 2, the absorbent member 4 has a rectangular shape that is long in the longitudinal direction X, and extends along the longitudinal direction X from the front portion A to the rear portion B. In the present embodiment, the respective positions of the absorbent member 4's both end portions in the longitudinal direction X substantially match the respective positions of the absorbent assembly 10's both end portions in the longitudinal direction X.

As illustrated in Fig. 2, a pair of leak-proof cuffs 6, 6 is provided respectively on both lateral sides along the longitudinal direction X on the skin-facing surface of the absorbent assembly 10. Each leak-proof cuff is formed by a liquid-resistant/water-repellent and air-permeable leak-proof-cuff-forming sheet 62. Each leak-proof cuff 6 includes one or more thread-shaped leak-proof-cuff-forming elastic members 61 provided along the longitudinal direction X in a stretched state. Each leak-proof cuff 6 stands up at least in the crotch portion C by the contraction of the elastic members 61 when the diaper 1 is worn. The leak-proof cuffs 6 are each formed by folding the continuous leak-proof-cuff-forming sheet 62 in two along its longitudinal direction, then fixing the leak-proof-cuff-forming elastic members 61 in their stretched state between opposing portions of the leak-proof-cuff-forming sheet, and joining together the opposing portions of the sheet.

The outer cover 5 forms the outer shape of the diaper 1 in its spread-open and stretched state as illustrated in Fig. 2. The peripheral edge of the outer cover 5 forms the contour line of the diaper 1 in the aforementioned state-i.e., forms the contour line in each of the front portion A, the crotch portion C, and the rear portion B. As illustrated in Fig. 2, in the crotch portion C, the outer cover 5's both lateral side edge portions, which extend along the outer cover's longitudinal direction X, are curved in an arc-shape protruding toward the central portion in the width direction Y, and thus, the outer cover 5's central region in the longitudinal direction X is narrowed inwardly in the width direction Y.

As illustrated in Figs. 2 and 3, the outer cover 5 includes: an outer sheet 51 that forms the outer surface, i.e., the non-skin-facing surface, of the diaper 1; an inner sheet 52 provided on the skin-facing surface side of the outer sheet 51; and a plurality of elastic members 55, 56, 57 provided between the two sheets 51, 52.

In the present embodiment, as illustrated in Fig. 2, the outer cover 5 includes: hip elastic members 55 located more outward, in the longitudinal direction X, than the absorbent assembly 10; and elastic members 56 located more inward than the absorbent assembly 10's both end portions in the longitudinal direction X. The elastic members 56 which are provided in positions overlapping the absorbent assembly 10 in the longitudinal direction X may be finely cut/separated or otherwise treated so as not to exhibit elasticity/extensibility in a region overlapping the absorbent assembly 10 in the thickness direction. The outer cover 5 includes, in each of the front portion A and the rear portion B, a plurality of thread-shaped or band-shaped elastic members 55, 56 arranged in the width direction Y in a stretched state, and the elastic members 55, 56 are arranged intermittently with predetermined intervals therebetween in the longitudinal direction X. By providing the elastic members 55, 56 in a state exhibiting elasticity/extensibility, the opening edge of the waist opening WH is provided with annular waist gathers (creases) which are substantially continuous over the entire circumference.

Referring to the outer cover 5, as illustrated in Figs. 1 and 2, in each leg edge portion LS forming the opening edge of the respective leg opening LH, at least one thread-shaped or band-shaped leg elastic member 57 for forming leg gathers is provided in a stretched state. Thus, annular leg gathers are formed substantially continuously over the entire circumference of the opening edge of each leg opening LH. The elastic members 55, 56, 57 in the outer cover 5 are sandwiched and fixed between the outer sheet 51 and the inner sheet 52, which constitute the outer cover 5, by a joining means such as an adhesive.

As illustrated in Fig. 3, the absorbent assembly 10 of the present embodiment includes elastic members 11a, 11b extending along the longitudinal direction X on both lateral sides, in the width direction Y, of the absorbent member 4. More specifically, the absorbent assembly includes, on the outside of each of the absorbent member 4's lateral sides in the width direction Y: a leak-proof-cuff-side elastic member 11a fixed in a stretched state between the leak-proof-cuff-forming sheet 62; and an absorbent-member-side elastic member 11b fixed in a stretched state between the absorbent member 4 and the topsheet 2. The end portions of these elastic members 11a, 11b are fixed in the front portion A and the rear portion B with an adhesive etc.

In the diaper 1 of the present embodiment, in addition to the contraction of the leak-proof-cuff-forming elastic members 61 along the longitudinal direction X, the leak-proof-cuff-side elastic member 11a and the absorbent-member-side elastic member 11b also contract in the longitudinal direction X. Thus, both lateral sides of the absorbent assembly 10 along the longitudinal direction X can stand up toward the skin-facing surface side more easily than the section located between the lateral sides. This configuration also contributes to making the pair of side absorbent members 4S, 4S of the absorbent member 4 stand up from the skin-facing surface side of the central absorbent member 4C.

As illustrated in Fig. 2, in the crotch portion C, the absorbent member 4 includes: a central absorbent member 4C extending in the longitudinal direction X; a pair of side absorbent members 4S, 4S respectively located on both sides, in the width direction Y, of the central absorbent member 4C, the side absorbent members configured to stand up from the skin-facing surface side of the central absorbent member 4C in a worn state; and a pair of bending-guide portions 45, 45, each bending-guide portion being formed between the central absorbent member 4C and the respective side absorbent member. Stated differently, the central absorbent member 4C and the respective side absorbent members 4S are divided by the respective bending-guide portions 45.

The side absorbent members 4S, 4S stand up as described above, due to deformation of the absorbent member 4, with the respective bending-guide portions 45 serving as base points, as will be described further below.

The side absorbent members 4S may be configured to stand up from the central absorbent member 4C in the later-described reference swollen state, or may be configured to stand up from the central absorbent member 4C even before the absorbent member 4 absorbs liquid.

For the sake of convenience of explanation, in Fig. 3, the side absorbent members 4S are illustrated in a state where they are not standing up.

The absorbent member 4 of the present embodiment includes an absorbent core 40, and a core-wrap sheet 48 covering the surface of the absorbent core 40. The absorbent core 40 includes absorbent materials, such as water-absorbent polymer 46, fiber material (not illustrated), etc. As illustrated in Fig. 2, the absorbent core 40 has a shape that is long in one direction. At least in the crotch portion C, the absorbent core 40 has a central region 41 in the central absorbent member 4C and side regions 43 in the respective side absorbent members 4S. The bending-guide portions 45 are interposed between the central region 41 and the respective side regions 43 in the width direction Y. That is, the absorbent core 40 in the crotch portion C is divided into the central region 41 and the side regions 43 by the pair of bending-guide portions 45, 45.

The bending-guide portion 45 is constituted either by a slit that penetrates the absorbent core 40 in the diaper 1's thickness direction Z, or a groove formed in the skin-facing surface of the absorbent core 40.

In the present embodiment, the bending-guide portion 45 is constituted by a slit, as illustrated in Fig. 3, and the core-wrap sheet 48 covering the absorbent core 40's skin-facing surface and the core-wrap sheet covering the absorbent core's non-skin-facing surface are joined together in the slit.

In the absorbent member 4 of the present embodiment, a single core-wrap sheet 48 collectively covers the central region 41 and the side regions 43.

In a diaper into which excretion fluid, such as urine, has been excreted, the absorbent core 40 swells as a result of absorbing excreted fluid. A model of a diaper in a state where excretion fluid has been excreted can be prepared according to the following method. First, an unused diaper (product) is brought into a spread-open and stretched state. Then, a mark (also referred to as "first mark") is made with an oil-based pen on the skin-facing surface of the topsheet 2 at the center position of the diaper 1, the center position being at the center in the longitudinal direction X and at the center in the width direction Y. Stated differently, the first mark is made at the intersection point between a width-direction central line that divides the diaper 1's entire length in the width direction Y equally in two and a longitudinal-direction central line that divides the diaper 1's entire length in the longitudinal direction X equally in two. Then, another mark is made at a position 7 cm away toward the front portion A side in the longitudinal direction X from the center position marked with the first mark, and the position marked with this other mark is employed as the injection point for injecting artificial urine. Next, a section forming the diaper 1's waist opening WH is gripped, and the diaper 1's crotch portion C is made to hang down such that the center position with the first mark is located at the lowest position. This state is referred to as "hanging-down state". In this hanging-down state, 160 g of artificial urine is injected to the injection point at an injection rate of 5 g/second. A tube pump can be used for this injection. Then, after injection of artificial urine, the diaper 1 is left to stand in the hanging-down state for 5 minutes. This state is referred to as "reference swollen state". Note that 160 g of artificial urine is equivalent to the amount of three to four times of urination by an infant/toddler.

The composition of the artificial urine is as follows: 1.94 mass% urea, 0.7954 mass% sodium chloride, 0.11058 mass% magnesium sulfate (heptahydrate), 0.06208 mass% calcium chloride (dihydrate), 0.19788 mass% potassium sulfate, 0.0035 mass% polyoxyethylene lauryl ether, and ion-exchanged water (balance).

Fig. 4 illustrates a cross-sectional view along the width direction Y of the absorbent member 4 in the reference swollen state. The absorbent core 40, which has swollen by absorbing liquid such as urine, has a longer width and greater thickness compared to a state before absorbing liquid. As a result, the width of each side region 43, as well as the width of the central region 41, becomes longer and the thickness becomes greater. In the present Description, "width" refers to the length in the width direction Y.

As illustrated in Fig. 4, in the reference swollen state, the width of the side region 43 is greater than the width of the central region 41.

Further, in the reference swollen state, half the width W1 of the central region 41 is less than the thickness t3 of each side region 43. More specifically, half the width W1 of the central region 41 is 0.7 times or less of the thickness t3 of each side region 43. In Fig. 4, reference sign Wa indicates "half the width W1 of the central region 41".

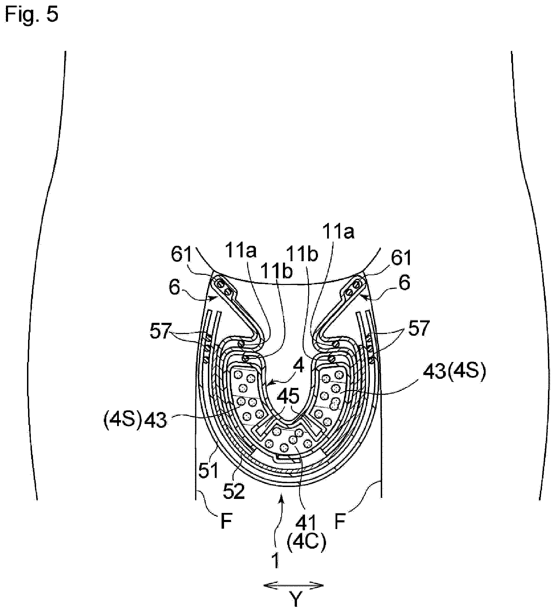

As illustrated in Fig. 5, in a worn state and swollen state after absorbing liquid such as urine, the diaper 1 of the present embodiment easily deforms in a U-shape in a cross-sectional view of the crotch portion C taken along the width direction Y. More specifically, the absorbent member 4 is bent/folded in a manner that the side regions 43-which are located respectively on both lateral sides, in the width direction Y, of the central region 41-stand up from the skin-facing surface side of the central region 41, employing the respective bending-guide portions 45, 45 as base points. Thus, the entire crotch portion C, and more specifically, the absorbent member 4 in the crotch portion C, can easily assume a U shape.

This deformation into a U shape occurs due to the side absorbent members 4S standing up, with the pair of bending-guide portions 45, 45 serving as axes of flexure.

In this absorbent member 4, the central region 41 constitutes the bottom portion of the U shape. Moreover, in the reference swollen state, the width of the side region 43 is longer than the width of the central region 41, and also, half the width W1 of the central region 41 is 0.7 times or less of the thickness t3 of each side region 43. Hence, the absorbent member 4 after deformation is easily fittable in a compact manner in the width direction Y, as illustrated in the cross section, and thus, the entire crotch portion C of the diaper 1 can be effectively suppressed from becoming bulky in the width direction Y. This effect is also achieved in a swollen state after absorbing liquid such as urine. Hence, with the diaper 1 of the present embodiment, the diaper 1 can be effectively suppressed from applying pressure to the wearer's femoral regions F, F, even in a swollen state, and thus, the wearer's movement is less likely to be restricted around the femoral regions F. Stated differently, even in a swollen state after absorbing liquid, the diaper 1 is less likely to hinder walking motion. Thus, the wearer of the diaper 1 can walk easily.

Since the bending-guide portions 45, 45 are formed between the central absorbent member 4C and the respective side absorbent members 4S, the bending-guide portions 45 can divide the central absorbent member 4C and the side absorbent members 4S from one another, and also divide the central region 41 and the side regions 43.

The width of the central region 41, as well as the width of each side region 43, does not include the width of the bending-guide portions 45 that divide these regions 41, 43. As will be described further below, in cases where the width of the absorbent core 40 varies in the crotch portion C, the "width of the central region 41" refers to the width at the narrowest part, where the width of the absorbent core 40 becomes the shortest. The width of the narrowest part where the width of the central region 41 becomes the shortest is defined as width W1 of the central region 41, and the width of the side region 43 on an extended line adjacent to the width W1 in the width direction is defined as W2. The width of the central region 41 and that of the side regions 43 in the reference swollen state are measured in the form of the absorbent member, wherein the absorbent core is covered by the core-wrap sheet.

The thickness of the central absorbent member 4C and that of the side absorbent member 4S in the reference swollen state, as well as the thickness of the central region 41 and that of the side region 43, are measured according to the following method.

The absorbent member 4 is taken out from the diaper 1 in the reference swollen state. The absorbent member 4 is placed on a horizontal place in a manner that there are no creases or folds and that the skin-facing surface faces upward in the vertical direction. Next, using a thickness gage, Intelligent-L LASER SENSOR (from Keyence Corporation), the thickness of the central absorbent member 4C and that of the side absorbent member 4S are measured under a load of 0.5 cN/cm². More specifically, the thickness of a section where the central region 41 is present in the central absorbent member 4C, or the thickness of a section where the side region 43 is present in the side absorbent member 4S, is measured. At this time, the thickness is measured by arranging, between the tip end portion of the thickness gage and the sample, a plate (an acrylic plate having a thickness of approximately 5 mm) having a circular or square planar-view shape and whose size has been adjusted such that the load is 0.5 cN/cm². At the time of measurement, in cases where the thickness of the central region 41 is small and the core-wrap sheet 48 in the central region 41 rises up from the absorbent core 40, the central absorbent member 4C is cut out from the absorbent member 4 along the bending-guide portions 45, 45, and the thickness of the central absorbent member 4C is measured in a state where the absorbent core 40 and the core-wrap sheet 48 within the central absorbent member 4C are in contact with one another. This measurement is performed at five arbitrary sites within a central range in the longitudinal direction for both the section where the central region 41 is present in the central absorbent member 4C and the section where the side region 43 is present in the side absorbent member 4S. The mean value of the measurement values for the five sites is considered to be the thickness of the central absorbent member 4C or the thickness of the side absorbent member 4S. A value found by subtracting the thickness of the core-wrap sheet 48 from the thickness of the central absorbent member 4C is considered to be the thickness of the central region 41, and a value found by subtracting the thickness of the core-wrap sheet 48 from the thickness of the side absorbent member 4S is considered to be the thickness of the side region 43. The aforementioned "central range in the longitudinal direction" is a range that passes through the center of the absorbent member 4 in the longitudinal direction X and that is within 5 cm on both sides in the longitudinal direction X from a lateral line extending in the width direction Y-i.e., a range within 5 cm in the longitudinal direction X toward both the front portion A side and the rear portion B side from the aforementioned lateral line.

In cases of removing a constituent member, such as the absorbent member etc., from the diaper, the constituent member is removed by first spraying the diaper with a cold spray to solidify the adhesive bonding the constituent members together, and then peeling off the constituent member from the other constituent members. This removal method is applicable in common to other measurement methods described in the present Description, unless specifically stated otherwise.

From the viewpoint of making the crotch portion C deform into a U shape that is more compact in the width direction Y and also reliably securing the absorption capacity of the absorbent core 40, it is preferable that the dimensions of the central region 41 and the side region 43 in the reference swollen state are within the following ranges.

The preferred dimensions of the side region 43 described below refer to the dimensions of a single side region 43, and not the total for the pair of side regions 43, 43.

In the reference swollen state, the ratio W2/W1 of W2 to W1, where W1 (see Fig. 4) is the central region 41's width and W2 (see Fig. 4) is the side region 43's width, is preferably 1.1 or greater, more preferably 1.5 or greater, and preferably 5 or less, more preferably 3 or less, and preferably from 1.1 to 5, more preferably from 1.5 to 3.

In the reference swollen state, the central region 41's width W1 (see Fig. 4) is preferably 8 mm or greater, more preferably 10 mm or greater, and preferably 35 mm or less, more preferably 30 mm or less, and preferably from 8 to 35 mm, more preferably from 10 to 30 mm.

In the reference swollen state, the side region 43's width W2 (see Fig. 4) is preferably 35 mm or greater, more preferably 40 mm or greater, and preferably 60 mm or less, more preferably 55 mm or less, and preferably from 35 to 60 mm, more preferably from 40 to 55 mm.

In the reference swollen state, the ratio t3/t2 of t3 to t2, where t2 (see Fig. 4) is the central region 41's thickness and t3 (see Fig. 4) is the side region 43's thickness, is preferably 0.5 or greater, more preferably 0.8 or greater, and preferably 2 or less, more preferably 1.5 or less, and preferably from 0.5 to 2, more preferably from 0.8 to 1.5.

In the reference swollen state, the central region 41's thickness t2 (see Fig. 4) is preferably 5 mm or greater, more preferably 10 mm or greater, and preferably 30 mm or less, more preferably 25 mm or less, and preferably from 5 to 30 mm, more preferably from 10 to 25 mm.

In the reference swollen state, the side region 43's thickness t3 (see Fig. 4) is preferably 10 mm or greater, more preferably 15 mm or greater, and preferably 30 mm or less, more preferably 25 mm or less, and preferably from 10 to 30 mm, more preferably from 15 to 25 mm.

From the same viewpoint, in the reference swollen state, Wa to t3, where Wa is half the width W1 of the central region 41 and t3 is the side region 43's thickness, is 0.7 times or less, and preferably 0.4 times or greater, more preferably 0.5 times or greater, and more preferably 0.67 times or less, and preferably from 0.4 to 0.7 times, more preferably from 0.5 to 0.67 times.

A test piece 1a, obtained by folding the diaper 1 in the reference swollen state along the longitudinal direction X in a manner that the diaper 1's width is divided equally in two, can be employed as a model of the diaper 1 located at the wearer's crotch section and having absorbed liquid such as urine.

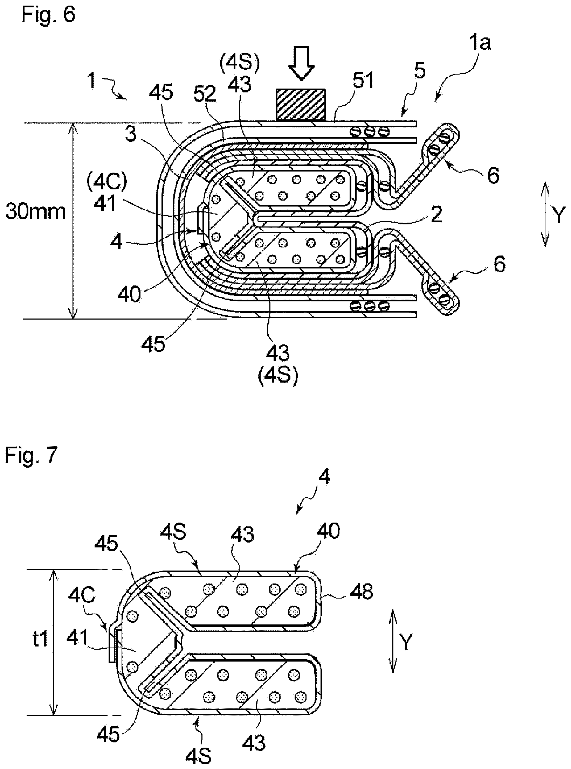

It is preferable that compression load to compress the test piece 1a until the test piece 1a's thickness in the crotch portion C becomes 30 mm (referred to hereinbelow as "30-mm compression load"), as illustrated in Fig. 6, is within the following range. The reason for employing 30-mm compression load as an index is based on the following findings. That is, the width at the wearer's crotch section is approximately 30 mm, with no significant difference between toddlers and adults; so when the width of the diaper in the crotch portion C is 30 mm, pressure from the diaper is less likely to be applied to the insides of both legs, and thus the diaper hardly interferes with nor affects the wearer's walking motion. It was also found that the amount of load for compressing the swollen-state diaper to 30 mm greatly affects the wearer's ease of walking.

From the viewpoint of making the diaper 1 deform more easily in the width direction Y and thereby further suppressing the diaper 1 in the reference swollen state from applying pressure to the femoral regions, it is preferable that the 30-mm compression load of the test piece 1a is preferably 7 N or less, more preferably 6.5 N or less, and preferably from 0.1 to 7 N, more preferably from 1 to 6.5 N.

The 30-mm compression load is measured according to the following method.

The diaper is brought into a spread-open and stretched state, and then brought into the reference swollen state. Then, the diaper is folded along the central line CL in the longitudinal direction so as to divide the width of the diaper equally in two, and this is employed as a test piece 1a. This test piece 1a is placed on a horizontal place in a manner that there are no creases or folds and that the longitudinal direction X matches the horizontal direction. A rectangular acrylic plate, being 5 cm wide and 15 cm long and weighing 28.7 g, is placed at the test piece 1a's center position in the longitudinal direction X (referred to hereinafter as "first measurement position") and also at a position located 2.5 cm away from the center position toward the front portion A side (referred to hereinafter as "second measurement position"). At this time, the acrylic plate is placed such that the acrylic plate's width matches the diaper's longitudinal direction X and such that the center of the plate's width matches the first measurement position or the second measurement position. Each acrylic plate is moved downward at a compression rate of 100 mm/minute, to compress the test piece 1a until its thickness becomes 30 mm. A material tester (e.g., Autograph AG-X from Shimadzu Corporation) is used for the compression. The compression load for when the diaper's thickness has become 30 mm is measured at each of the first measurement position and the second measurement position, and the mean value of the two points is considered to be the 30-mm compression load.

It has been verified that there is a correlation between the 30-mm compression load and compression load to compress the side absorbent member 4S in the reference swollen state until the thickness thereof becomes 10 mm (referred to hereinafter as "10-mm compression load"). Stated differently, by adjusting the 10-mm compression load of the side absorbent member 4S, it is possible to set the 30-mm compression load of the test piece 1a within the aforementioned range.

From this viewpoint, it is preferable that the 10-mm compression load of at least one of the side absorbent members 4S is preferably 4.5 N or less, more preferably 4 N or less, and preferably from 0.1 to 4.5 N, more preferably from 0.5 to 4 N.

It is preferable that the 10-mm compression load of both the side absorbent members 4S, 4S is within the aforementioned range.

The method for measuring the 10-mm compression load of the side absorbent member 4S is as follows. First, the absorbent member 4 is removed from a diaper in the reference swollen state, and the absorbent member 4 is placed on a horizontal place in a manner that there are no creases or folds and that its skin-facing surface faces upward in the vertical direction. Next, using a material tester (e.g., Autograph AG-X from Shimadzu Corporation) equipped with a 2-cm-dia. rod-shaped compression test jig, the compression load to compress the side absorbent member 4S until its thickness becomes 10 mm is measured. This measurement is performed at three arbitrary sites in a section where the absorbent core 40 is present in the side absorbent member 4S, and the mean value thereof is considered to be the 10-mm compression load.

From the viewpoint of making the 10-mm compression load of the side absorbent member 4S fall within the aforementioned range more easily, it is preferable that, when the absorbent member 4, in the reference swollen state, is folded along the longitudinal direction in a manner that the absorbent member 4's width is divided equally in two, the thickness t1 (see Fig. 7) of the absorbent member 4 in the crotch portion C is preferably 20 mm or greater, more preferably 25 mm or greater, and preferably 60 mm or less, more preferably 50 mm or less, and preferably from 20 to 60 mm, more preferably from 25 to 50 mm.

This thickness is measured according to the same method as the aforementioned method for measuring the absorbent member's thickness, except that the sample is folded such that its width is divided equally in two. In this measurement, the aforementioned plate having a circular or square planar-view shape is placed horizontally.

From the viewpoint of making the crotch portion C deform easily into the aforementioned U shape and further suppressing the crotch portion C from getting bulky in the width direction Y, it is preferable that the basis weight of the side region 43 is equal to or greater than the basis weight of the central region 41.

From the same viewpoint, it is preferable that the basis weight of the side region 43 and that of the central region 41 are within the following ranges. The basis weight is measured in a state before absorbing liquid. In the following description, the preferred basis weight of the side region 43 refers to the basis weight of a single side region 43, and not the total basis weight for the pair of side regions 43, 43.

The ratio B2/B1 of B2 to B1, where B1 is the central region 41's basis weight and B2 is the side region 43's basis weight, is preferably 1 or greater, more preferably 1.1 or greater, and preferably 3 or less, more preferably 2 or less, and preferably from 1 to 3, more preferably from 1.1 to 2.

The central region 41's basis weight B1 is preferably 80 g/m² or greater, more preferably 100 g/m² or greater, and preferably 650 g/m² or less, more preferably 450 g/m² or less, and preferably from 80 to 650 g/m², more preferably from 100 to 450 g/m².

The side region 43's basis weight B2, on condition that it is equal to or greater than the central region 41's basis weight, is preferably 80 g/m² or greater, more preferably 110 g/m² or greater, and preferably 700 g/m² or less, more preferably 500 g/m² or less, and preferably from 80 to 700 g/m², more preferably from 110 to 500 g/m².

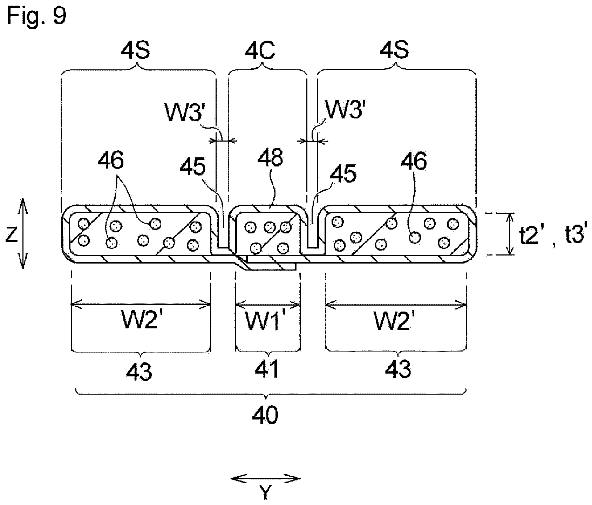

From the viewpoint of making the crotch portion C deform easily into the aforementioned U shape, with the bending-guide portions 45 serving as base points, it is preferable that the width W3' (see Fig. 9) of each bending-guide portion 45 before absorbing liquid is preferably 10 mm or greater, more preferably 13 mm or greater, and preferably 30 mm or less, more preferably 20 mm or less, and preferably from 10 to 30 mm, more preferably from 13 to 20 mm.

In the absorbent member 4 of the present embodiment, before absorbing liquid, the side absorbent member 4S's thickness and the central absorbent member 4C's thickness are substantially the same. This configuration is preferable from the viewpoint of suppressing the formation of large projections and depressions on the surface of the diaper before absorbing liquid, and thereby maintaining a more favorable appearance.

In the present embodiment, in the reference swollen state, the side regions 43 swell more than the central region 41, and hence, the thickness of the side absorbent member 4S becomes greater than the thickness of the central absorbent member 4C. Instead of this configuration, the absorbent member 4 may be configured such that, before absorbing liquid, the thickness of the side absorbent members 4S is different from the thickness of the central absorbent member 4C.

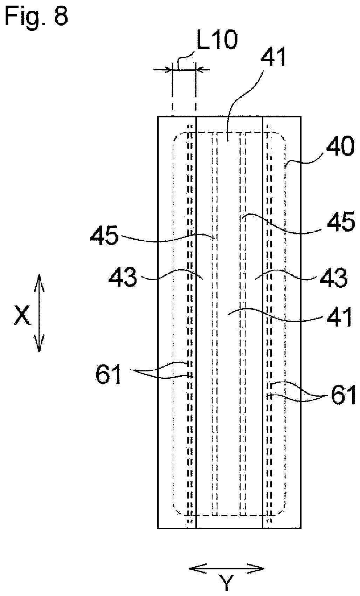

The absorbent member 4 only needs to have the central absorbent member 4C, the side absorbent members 4S and the bending-guide portions 45 at least in the crotch portion C, and one or more of these members 41, 43, 45 may extend up to one or both the absorbent member 4's front portion A and the absorbent member 4's rear portion B. In the present embodiment, as illustrated in Fig. 8, the absorbent member 4 includes the central absorbent member 4C, the side absorbent members 4S and the bending-guide portions 45 over the entire length in the longitudinal direction X.

As illustrated in Fig. 8, the central region 41 in the present embodiment is formed rectilinearly, extends over the entire length of the absorbent core 40 in the longitudinal direction, and has the same width over its entire length. Stated differently, the pair of bending-guide portions 45, 45 extends rectilinearly in the longitudinal direction X parallel to one another over the entire length of the absorbent core 40, and thereby, the width of the central region 41 is constant over the entire length of the absorbent core 40 in the longitudinal direction. With this configuration, the lateral side edges of the diaper 1, which has assumed the reference swollen state and whose cross-sectional shape has been deformed into the U shape, is less likely to apply pressure to the wearer's femoral regions, and thus, the range of motion of the wearer's femoral regions can be increased. As a result, movements, such as large rotation of the femoral region about the femoral axis, can be facilitated.

From the viewpoint of reducing the area covered by the leak-proof cuffs 6 and thereby further facilitating swelling of the side regions 43, it is preferable that the length L10 (see Fig. 8) of the section where the leak-proof cuff 6 overlaps the absorbent core 40 in the width direction Y is shorter than the width W2' (see Fig. 9) of the side region 43 before absorbing liquid.

From the same viewpoint, it is preferable that the length L10 of the section where the leak-proof cuff 6 overlaps the absorbent core 40 in the width direction Y and the width W2' of the side region 43 before absorbing liquid are within the following ranges.

The length L10 of the section where the leak-proof cuff 6 overlaps the absorbent core 40 in the width direction Y is measured in the diaper 1 in a spread-open and stretched state and before absorbing liquid.

The ratio L10/W2' of L10 to W2', where L10 is the length of the section where the leak-proof cuff 6 overlaps the absorbent core 40 in the width direction Y and W2' is the side region 43's width before absorbing liquid, is preferably 0.1 or greater, more preferably 0.15 or greater, and preferably 0.7 or less, more preferably 0.6 or less, and preferably from 0.1 to 0.7, more preferably from 0.15 to 0.6.

The length L10 of the section where the leak-proof cuff 6 overlaps the absorbent core 40 in the width direction Y is preferably 5 mm or greater, more preferably 8 mm or greater, and preferably 40 mm or less, more preferably 30 mm or less, and preferably from 5 to 40 mm, more preferably from 8 to 30 mm.

The side region 43's width W2' before absorbing liquid is preferably 30 mm or greater, more preferably 35 mm or greater, and preferably 55 mm or less, more preferably 50 mm or less, and preferably from 30 to 55 mm, more preferably from 35 to 50 mm.

Due to swelling of the central region 41 and the side regions 43 by the absorption of liquid, the width W3 (see Fig. 4) of the bending-guide portion 45 in the reference swollen state is likely to become shorter than that before absorbing liquid. Stated differently, the space within the bending-guide portions 45 tends to be closed up by the swollen central region 41 and the side regions 43.

From the viewpoint of allowing the aforementioned U shape of the crotch portion C to be maintained more stably, it is preferable that, even in the reference swollen state, the absorbent core 40 is maintained in a state where the central region 41 and the side regions 43 are divided from one another.

From this viewpoint, in cases where the bending-guide portion 45 is constituted by a slit, it is preferable that the core-wrap sheet 48 located on the absorbent core 40's skin-facing surface side and the core-wrap sheet located on the absorbent core's non-skin-facing surface side are joined together in the slit, and more preferable that the core-wrap sheet 48 on the skin-facing surface side and the core-wrap sheet on the non-skin-facing surface side are joined together also in the reference swollen state (see Fig. 4).

From the same viewpoint, it is preferable that the joining strength between the core-wrap sheet 48 covering the absorbent core 40's skin-facing surface and the core-wrap sheet covering the absorbent core's non-skin-facing surface in the bending-guide portion 45, constituted by a slit, is preferably 5 cN/10 mm or greater, more preferably 100 cN/10 mm or greater. The method for measuring the joining strength is as follows.

### Method for Measuring Joining Strength:

In an environment at 22°C and 65% RH, the absorbent member is removed from an unused disposable diaper and is used as a measurement sample. Next, at the bending-guide portion in the absorbent core of the measurement sample, the core-wrap sheet covering the absorbent core's skin-facing surface and the core-wrap sheet covering the absorbent core's non-skin-facing surface, which are joined together, are attached to a tensile tester (e.g., Tensilon tensile tester RTA-100 from Orientec Co., Ltd.). For example, the core-wrap sheet covering the absorbent core's skin-facing surface is attached to the upper chuck of the tensile tester, and the core-wrap sheet covering the absorbent core's non-skin-facing surface is attached to the lower chuck. The distance between the upper and lower chucks is set to 10 mm. Next, with the position of the lower chuck fixed, the upper chuck is raised at a speed of 300 mm/min, to peel apart the core-wrap sheet(s) joined in the bending-guide portion. At this time, tensile load that changes with the rise of the upper chuck-i.e., that changes with tensile distance-is measured. In a tensile distance-tensile load curve obtained by this measurement, the first maximum point, which appears first from the start of measurement, is found as the tensile load. This measurement is repeated three times, and the mean value thereof is considered to be the joining strength between the core-wrap sheet(s).

In the present embodiment, the absorbent core 40 including the pair of bending-guide portions 45, 45 can be manufacturing using a known fiber-stacking device. The fiber-stacking device is a device that typically includes a rotary drum having an accumulation depression in the outer circumferential surface thereof, wherein: core-forming materials are supplied to the drum's outer circumferential surface in a dispersed and airborne state while rotating the rotary drum, and the core-forming materials are stacked in the accumulation depression by suction from the bottom surface of the accumulation depression; and the fiber stack within the accumulation depression is then released from the accumulation depression by suction by a suction means arranged in opposition to the accumulation depression, and is transferred onto the suction means. In the thus-configured fiber-stacking device, a portion of the air-permeable bottom surface of the accumulation depression is made air-impermeable or sparingly air-permeable by, for example, arranging an air-impermeable member or a sparingly air-permeable member on a portion of the bottom surface. In this way, the core-forming materials are less prone to get stacked on the air-impermeable or sparingly air-permeable portion at the time of stacking the core-forming materials, and thus, the amount of core-forming materials stacked on the air-impermeable or sparingly air-permeable portion becomes smaller compared to other sections of the bottom surface. By providing such air-impermeable or sparingly air-permeable portions, it is possible to form an absorbent core having a pair of bending-guide portions 45, 45 between the central region 41 and the side regions 43. Hence, by using such a fiber-stacking device having a rotary drum in which a portion of the bottom surface of the accumulation depression is made air-impermeable or sparingly air-permeable, sections corresponding to the air-impermeable or sparingly air-permeable portions become the bending-guide portions 45 constituted by a slit or a groove, whereas sections corresponding to the other sections of the bottom surface become the central region 41 and the side regions 43. In this way, the absorbent core 40 of the present embodiment can be manufactured.

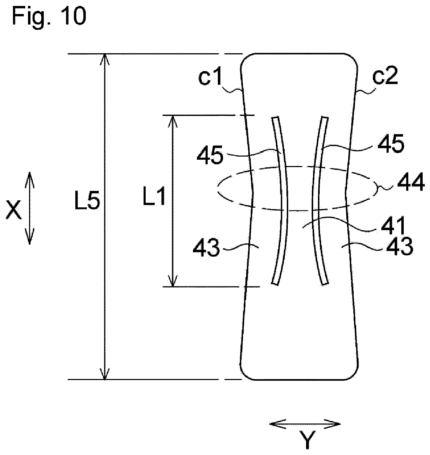

Next, other embodiments of the disposable diaper of the present invention will be described with reference to the drawings. The explanation on the foregoing embodiment applies as appropriate to the other embodiments, unless there are contradictions. In Figs. 10 to 12, constituent elements that are the same as those in the foregoing embodiment are accompanied by the same reference signs as in the foregoing embodiment. The following explanation on the embodiments illustrated in Figs. 10 to 12 applies to a state of the absorbent core 40 before absorbing liquid, unless specifically stated otherwise as "reference swollen state".

In the absorbent core 40 of the foregoing embodiment, both lateral side edges along the longitudinal direction X are rectilinear and are parallel to the longitudinal direction X over the entire length of the absorbent core (see Fig. 8). Instead, the absorbent core may have a width that varies along the longitudinal direction X.

For example, the absorbent core 40c illustrated in Fig. 10 has a narrowed portion 44 where both lateral side edges c1, c2 of the absorbent core extending along the longitudinal direction X are narrowed inwardly in the width direction Y.

From the viewpoint of further facilitating the motion of moving the femoral region forward and further facilitating walking motion, it is preferable that the absorbent core 40c has the narrowed portion 44 located more toward the front portion A side than the center in the longitudinal direction X. From the same viewpoint, it is preferable that the narrowed portion 44 is located in the crotch portion C of the diaper 1, and more preferably located more toward the front portion A side than the center of the crotch portion C in the longitudinal direction X.

The absorbent core 40c illustrated in Fig. 10 includes a central region 41 whose length is shorter than the entire length of the absorbent core 40 in the longitudinal direction X.

From the viewpoint of further facilitating deformation of the diaper 1 in the crotch portion C, it is preferable that the length L1 (see Fig. 10) of the central region 41 in the longitudinal direction X and the length L5 (see Fig. 10) of the absorbent core 40c in the longitudinal direction X are within the following ranges.

It is preferable that the ratio L1/L5 of L1 to L5, where L1 (see Fig. 10) is the central region 41's length in the longitudinal direction X and L5 (see Fig. 10) is the absorbent core 40c's length in the longitudinal direction X, is preferably 0.3 or greater, more preferably 0.5 or greater.

The central region 41's length L1 (see Fig. 10) in the longitudinal direction X is preferably 60 mm or greater, more preferably 100 mm or greater, and preferably 550 mm or less, more preferably 500 mm or less, and preferably from 60 to 550 mm, more preferably from 100 to 500 mm.

The absorbent core 40c's length L5 (see Fig. 10) in the longitudinal direction X is, on condition that it is longer than the length L1 of the central region 41 in the longitudinal direction X, preferably 250 mm or greater, more preferably 300 mm or greater, and preferably 800 mm or less, more preferably 700 mm or less, and preferably from 250 to 800 mm, more preferably from 300 to 700 mm.

The central region 41 illustrated in Fig. 10 has a width that varies along the longitudinal direction X. More specifically, each of the bending-guide portions 45, 45, which extend along the longitudinal direction X, is formed curvilinearly, and, in the vicinity of the center in the longitudinal direction X, each bending-guide portion is curved inwardly in the width direction Y. Thus, the central region 41 has a shape that is narrowed inwardly in the width direction Y in the vicinity of the center in the longitudinal direction X. The bending-guide portion 45 illustrated in Fig. 10 has the same width along the longitudinal direction X.

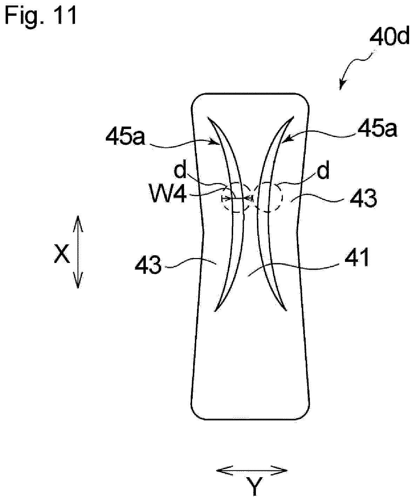

The absorbent core 40d illustrated in Fig. 11 has the same configuration as that illustrated in Fig. 10, except that each bending-guide portion 45a has a width that varies along the longitudinal direction X.

Each of the bending-guide portions 45a illustrated in Fig. 11 has a width that varies along the longitudinal direction X, and has a maximum width portion d where the width becomes the greatest, the maximum width portion d being located more toward the front portion A side than the center in the longitudinal direction X. With this configuration, a section of the absorbent core 40d more toward the front portion A side than the center in longitudinal direction X can be easily bent into the aforementioned U shape, thereby further facilitating the motion of moving the femoral region forward.

From the viewpoint of achieving this effect more reliably, it is preferable that the width W4 (see Fig. 11) of the maximum width portion d of the bending-guide portion 45a is preferably 10 mm or greater, more preferably 12 mm or greater, and preferably 30 mm or less, more preferably 20 mm or less, and preferably from 10 to 30 mm, more preferably from 12 to 20 mm.

The shape of the contour of the bending-guide portion 45 may include rectilinear portions as illustrated in Fig. 8, or may include curvilinear portions as illustrated in Figs. 10 and 11. Alternatively, the shape of the contour of the bending-guide portion 45 may include both rectilinear portions and curvilinear portions.

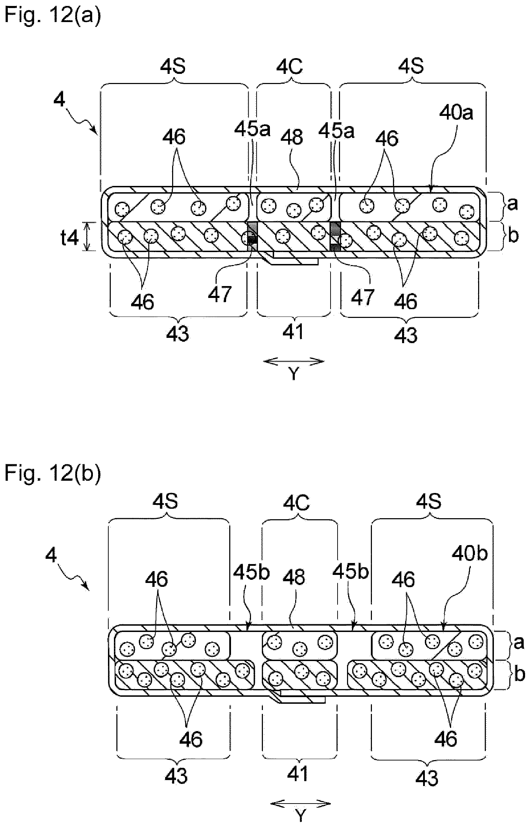

As illustrated in Fig. 12, the absorbent core 40 may have a layered structure in which layers made of absorbent materials are stacked. In Fig. 12, in the central region 41 and the side regions 43, the absorbent core 40 has a structure wherein two layers of absorbent materials are stacked. On the other hand, no layer of absorbent materials is present in the bending-guide portions 45. Such a layered structure is preferable, in terms that the central region 41 and the side regions 43 are easy to form, and that the absorbent member 4 can bend more easily at the bending-guide portions 45 serving as base points.

Examples of absorbent cores 40 having a layered structure may include absorbent cores 40a, 40b, as illustrated in Figs. 12(a) and 12(b), each having a structure with two layers, a and b, in both the central region 41 and the side regions 43.

The bending-guide portion 45a illustrated in Fig. 12(a) is constituted by a groove formed such that the aforementioned layer a is not present on the skin-facing surface side.

The bending-guide portion 45b illustrated in Fig. 12(b) is constituted by: a slit formed such that the two layers a, b are not present on either the skin-facing surface side or the non-skin-facing surface side; and a groove whose bottom portion is constituted by a section formed by the layer b on the non-skin-facing surface side extending out in the width direction from the layer a on the skin-facing surface side.

The bending-guide portion 45a illustrated in Fig. 12(a) is constituted by a groove, as described above. The absorbent core 40a of the present embodiment has a thin portion 47 located on the non-skin-facing surface side of the bending-guide portion 45a, which is constituted by a groove, and between the central region 41 and the side region 43 in the width direction Y. Stated differently, the thin portion 47 forms the bottom portion of the groove, which is the bending-guide portion 45a.

The thin portion 47 in the embodiment illustrated in Fig. 12(a) is constituted by the layer b on the non-skin-facing surface side forming the absorbent core 40a.

From the viewpoint of making the absorbent member 4 bend more easily into the aforementioned U shape, it is preferable that the thin portion 47's basis weight is preferably 1 g/m² or greater, and preferably 120 g/m² or less, more preferably 115 g/m² or less, and preferably from 1 to 120 g/m², more preferably from 1 to 115 g/m².

From the same viewpoint, in the reference swollen state, the ratio t3/t4 of t3 to t4, where t3 is the side region 43's thickness and t4 is the thin portion 47's thickness, is preferably 2 or greater, more preferably 3 or greater, and preferably 15 or less, more preferably 10 or less, and preferably from 2 to 15, more preferably from 3 to 10.

From the same viewpoint, in the reference swollen state, the thin portion 47's thickness t4 (see Fig. 12(a)) is preferably 1 mm or greater, more preferably 3 mm or greater, and preferably 10 mm or less, more preferably 8 mm or less, and preferably from 1 to 10 mm, more preferably from 3 to 8 mm.

From the viewpoint of making the absorbent member 4 bend more easily in the reference swollen state, it is preferable that the absorbent member 4 includes a bending-guide portion other than the pair of bending-guide portions 45, 45. The other bending-guide portion is constituted by a slit or groove, as described above. The width of the other bending-guide portion is included within the width of the central region 41 or the side region 43 including the other bending-guide portion.

From the same viewpoint, it is preferable that the other bending-guide portion is formed in a central portion, in the width direction Y, of the central region 41 including the center of the central region 41, or in a central portion, in the width direction Y, of the side region 43 including the center of the side region 43.

In cases where the absorbent core 40 includes other bending-guide portions, it is preferable that the bending-guide portions 45, 45 are formed at positions symmetric with one another with respect to the longitudinal central line CL, and are the two bending-guide portions closest to the longitudinal central line CL. It is preferable that each of the bending-guide portions 45, 45 has a width that is greater than that of the other bending-guide portion(s).

Materials for forming the various parts of the disposable diapers of the foregoing embodiments are described below.

As for absorbent materials primarily constituting the absorbent core 40, any material conventionally used as a material for an absorbent member in this type of absorbent article may be used without particular limitation, with examples including wood pulp, hydrophilized synthetic fibers, water-absorbent polymers, etc. A typical form of the absorbent core may be, for example, a fiber aggregate of hydrophilic fibers such as wood pulp, or a member in which water-absorbent polymer particles are retained in the aforementioned fiber aggregate.

In the diaper 1, the core-wrap sheet 48 covers the entire region of the absorbent core's skin-facing surface and non-skin-facing surface. The core-wrap sheet 48 may be a single continuous sheet, or may include a single skin-side core-wrap sheet which covers the absorbent core 40's skin-facing surface and a single non-skin-side core-wrap sheet which is separate from the skin-side core-wrap sheet and covers the absorbent core 40's non-skin-facing surface. For the core-wrap sheet 48, it is possible to use, for example, a liquid-permeable sheet such as paper, one of various types of nonwoven fabrics, a porous film, etc. The absorbent core and the core-wrap sheet may be joined together by a known joining means such as a hot-melt adhesive.

For sheets constituting the outer cover 5, such as the outer sheet 51, the inner sheet 52, etc., it is possible to use nonwoven fabrics made by various manufacturing methods, with examples including spunbond nonwoven fabrics, air-through nonwoven fabrics, spun-laced nonwoven fabrics, heat-rolled nonwoven fabrics, meltblown nonwoven fabrics, or laminated nonwoven fabrics of the above.

For the various elastic members 11a, 11b, 61, 55, 56, 57 described above, it is possible to use, for example, synthetic rubber such as styrene-butadiene, butadiene, isoprene or neoprene, natural rubber, EVA, extensible polyolefins, or polyurethane. As for the shapes of the elastic members, it is possible to preferably use thread-form members (rubber threads) having a rectangular, square, circular, elliptic, or polygonal cross-sectional shape, or band-form members (rubber bands), or multifilament-type thread-form members.

The present invention has been described above according to preferred embodiments thereof, but the present invention is not limited to the foregoing embodiments and can be modified as appropriate. Also, the foregoing embodiments may be employed in combination.

For example, as illustrated in Fig. 2, the aforementioned diaper 1 is a pull-on disposable diaper including an outer cover 5 having an hourglass-like shape in which the front portion A, the crotch portion C and the rear portion B are continuous. The diaper may, however, be a separate-type pull-on disposable diaper wherein the outer cover 5 is divided into a front-side outer cover, a rear-side outer cover and a crotch outer cover as separate members. The disposable diaper of the present invention is not limited to a pull-on disposable diaper, and may be an open-type disposable diaper. Further, the disposable diaper of the present invention may be a disposable diaper either for toddlers or adults.

In the foregoing embodiments, the diaper 1 includes leak-proof-cuff-forming elastic members 61 and elastic members 11a, 11b located outside the absorbent member 4 in the width direction Y. However, it will suffice if the diaper includes either one of these elastic members 61, 11a, 11b, or includes two or more of these elastic members.

In the foregoing embodiment illustrated in Fig. 4, the core-wrap sheet 48 located on the absorbent core 40's skin-facing surface side and the core-wrap sheet located on the absorbent core's non-skin-facing surface side are joined together in the bending-guide portion 45 constituted by a slit. The core-wrap sheets, however, do not have to be joined in the bending-guide portion 45, as illustrated in Fig. 12(b).

In the foregoing embodiment illustrated in Fig. 12(a), the layer constituting the central region 41 is continuous with the layer on the non-skin-facing surface side in the side regions 43. Note, however, that the layer constituting the central region 41 and the layer on the non-skin-facing surface side in the side regions 43 may be formed by layers that are separate from one another.

In the foregoing embodiments, each leak-proof cuff 6 is formed by folding the leak-proof-cuff-forming sheet 62 in two. The leak-proof cuff, however, may be formed such that the leak-proof-cuff-forming sheet 62 is folded back only in the section where the leak-proof-cuff-forming elastic members 61 are to be fixed, and other sections are constituted by a single sheet.

In relation to the foregoing embodiments of the present invention, the present invention further discloses the following disposable diapers.
{1} A disposable diaper comprising a topsheet, a backsheet, and an absorbent member arranged between the topsheet and the backsheet,
   the absorbent member including an absorbent core,
   the disposable diaper having a longitudinal direction and a width direction orthogonal to the longitudinal direction,
   the disposable diaper having a rear portion to be arranged on a wearer's rear side when worn, a front portion to be arranged on the wearer's front side when worn, and a crotch portion located between the front portion and the rear portion, wherein:
      in the crotch portion, the absorbent member includes
         a central absorbent member extending in the longitudinal direction,
         a pair of side absorbent members respectively located on both sides, in the width direction, of the central absorbent member, the side absorbent members being configured to stand up from a skin-facing surface side of the central absorbent member in a worn state, and
         a pair of bending-guide portions, each of the bending-guide portions being formed between the central absorbent member and the respective side absorbent member;
      the absorbent core has a central region in the central absorbent member and side regions in the respective side absorbent members, the central region and the side regions being divided by the pair of bending-guide portions;
      the bending-guide portion is constituted by a slit or a groove;
      a width of the bending-guide portion before absorbing liquid is from 10 to 30 mm;
      in a reference swollen state achieved by injecting 160 g of artificial urine to an injection point located 7 cm away toward the front portion side in the longitudinal direction from a center position of the disposable diaper, which is at a center in the longitudinal direction and at a center in the width direction, and leaving the disposable diaper to stand for 5 minutes, a width of the side region of the absorbent core is greater than a width of the central region; and
      in the reference swollen state, half the width of the central region is 0.7 times or less of a thickness of each said side region.
{2} The disposable diaper as set forth in clause {1}, wherein, in a test piece obtained by folding the disposable diaper in the reference swollen state along the longitudinal direction in a manner that the disposable diaper's width is divided equally in two, compression load to compress the test piece until the test piece's thickness in the crotch portion becomes 30 mm is 7 N or less, preferably 6.5 N or less, and preferably from 0.1 to 7 N, more preferably from 1 to 6.5 N.
{3} The disposable diaper as set forth in clause {1} or {2}, wherein, in the reference swollen state, compression load to compress at least one of the pair of side absorbent members until the thickness thereof becomes 10 mm is 4.5 N or less, preferably 4 N or less, and preferably from 0.1 to 4.5 N, more preferably from 0.5 to 4 N.
{4} The disposable diaper as set forth in any one of clauses {1} to {3}, wherein, when the absorbent member is folded in the reference swollen state along the longitudinal direction in a manner that the absorbent member's width is divided equally in two, the absorbent member's thickness in the crotch portion is 60 mm or less, preferably from 20 to 60 mm, more preferably from 25 to 50 mm.
{5} The disposable diaper as set forth in any one of clauses {1} to {4}, wherein the absorbent core has a two-layer structure.
{6} The disposable diaper as set forth in any one of clauses {1} to {5}, wherein the side region's basis weight is equal to or greater than the central region's basis weight.
{7} The disposable diaper as set forth in any one of clauses {1} to {6}, wherein a ratio B2B1 of B2 to B1 is from 1 to 3, preferably from 1.1 to 2, where B1 is the central region's basis weight and B2 is the side region's basis weight.
{8} The disposable diaper as set forth in any one of clauses {1} to {7}, wherein the central region's basis weight is from 80 to 650 g/m², preferably from 100 to 450 g/m².
{9} The disposable diaper as set forth in any one of clauses {1} to {8}, wherein the side region's basis weight is from 80 to 700 g/m², preferably from 110 to 500 g/m².
{10} The disposable diaper as set forth in any one of clauses {1} to {9}, wherein, in the reference swollen state, a ratio W2/W1 of W2 to W1 is from 1.1 to 5, preferably from 1.5 to 3, where W1 is the central region's width and W2 is the side region's width.
{11} The disposable diaper as set forth in any one of clauses {1} to {10}, wherein, in the reference swollen state, the central region's width is from 8 to 35 mm, preferably from 10 to 30 mm.
{12} The disposable diaper as set forth in any one of clauses {1} to {11}, wherein, in the reference swollen state, the side region's width is from 35 to 60 mm, preferably from 40 to 55 mm.
{13} The disposable diaper as set forth in any one of clauses {1} to {12}, wherein the width of the bending-guide portion before absorbing liquid is from 13 to 20 mm.
{14} The disposable diaper as set forth in any one of clauses {1} to {13}, wherein, in the reference swollen state, a ratio t3/t2 of t3 to t2 is from 0.5 to 2, preferably from 0.8 to 1.5, where t2 is the central region's thickness and t3 is the side region's thickness.
{15} The disposable diaper as set forth in any one of clauses {1} to {14}, wherein, in the reference swollen state, the central region's thickness is from 5 to 30 mm, preferably from 10 to 25 mm.
{16} The disposable diaper as set forth in any one of clauses {1} to {15}, wherein, in the reference swollen state, the side region's thickness is from 10 to 30 mm, preferably from 15 to 25 mm.
{17} The disposable diaper as set forth in any one of clauses {1} to {16}, wherein, in the reference swollen state, the ratio of half the central region's width to the side region's thickness is from 0.4 to 0.7 times, preferably from 0.5 to 0.67 times.
{18} The disposable diaper as set forth in any one of clauses {1} to {17}, wherein the absorbent member includes a bending-guide portion other than the pair of bending-guide portions.
{19} The disposable diaper as set forth in any one of clauses {1} to {18}, wherein the absorbent core has a narrowed portion where both lateral side edges of the absorbent core extending along the longitudinal direction are narrowed inwardly in the width direction, the narrowed portion being located more toward the front portion side than the center in the longitudinal direction.
{20} The disposable diaper as set forth in any one of clauses {1} to {19}, wherein: the width of each said bending-guide portion varies along the longitudinal direction; and
   each said bending-guide portion has a maximum width portion where the width becomes the greatest, the maximum width portion being located more toward the front portion side than the center in the longitudinal direction.
{21} The disposable diaper as set forth in clause {20}, wherein the maximum width portion's width is from 10 to 30 mm, preferably from 12 to 20 mm.
{22} The disposable diaper as set forth in any one of clauses {1} to {21}, wherein the bending-guide portion is formed rectilinearly.
{23} The disposable diaper as set forth in any one of clauses {1} to {22}, wherein the bending-guide portion is formed curvilinearly.
{24} The disposable diaper as set forth in any one of clauses {1} to {23}, further comprising an absorbent assembly including the topsheet and the absorbent member, and further including a pair of leak-proof cuffs on respective lateral sides extending along the longitudinal direction,
   wherein, in the width direction, a length of a section where the leak-proof cuff overlaps the absorbent core is shorter than the side region's width before absorbing liquid.
{25} The disposable diaper as set forth in clause {24}, wherein a ratio L10/W2' of L10 to W2' is from 0.1 to 0.7, preferably from 0.15 to 0.6, where L10 is the length of the section where the leak-proof cuff overlaps the absorbent core in the width direction and W2' is the side region's width before absorbing liquid.
{26} The disposable diaper as set forth in clause {24} or {25}, wherein the length of the section where the leak-proof cuff overlaps the absorbent core in the width direction is from 5 to 40 mm, preferably from 8 to 30 mm.
{27} The disposable diaper as set forth in any one of clauses {24} to {25}, wherein the side region's width in the width direction before absorbing liquid is from 30 to 55 mm, preferably from 35 to 50 mm.
{28} The disposable diaper as set forth in any one of clauses {1} to {27}, wherein:
   the absorbent member includes a core-wrap sheet covering the absorbent core;
   the bending-guide portion is constituted by a slit; and
   the core-wrap sheet located on the absorbent core's skin-facing surface side and the core-wrap sheet located on the absorbent core's non-skin-facing surface side are joined together in the slit.
{29} The disposable diaper as set forth in clause {28}, wherein the core-wrap sheet on the skin-facing surface side and the core-wrap sheet on the non-skin-facing surface side are joined together also in the reference swollen state.
{30} The disposable diaper as set forth in clause {28} or {29}, wherein a joining strength between the core-wrap sheet is 5 cN/10 mm or greater, preferably 100 cN/10 mm or greater.
{31} The disposable diaper as set forth in any one of clauses {1} to {30}, wherein:
   the bending-guide portion is constituted by a groove;
   the absorbent core has a thin portion located on the groove's non-skin-facing surface side and between the central region and each said side region; and
   the thin portion's basis weight is from 1 to 120 g/m², preferably from 1 to 115 g/m2.
{32} The disposable diaper as set forth in clause {31}, wherein, in the reference swollen state, a ratio t3/t4 of t3 to t4 is 2 or greater, preferably from 2 to 15, more preferably from 3 to 10, where t3 is the side region's thickness and t4 is the thin portion's thickness.
{33} The disposable diaper as set forth in clause {32}, wherein the thin portion's thickness is from 1 to 10 mm, preferably from 3 to 8 mm.
{34} The disposable diaper as set forth in any one of clauses {1} to {33}, wherein a ratio L1/L5 of L1 to L5 is preferably 0.3 or greater, preferably 0.5 or greater, where L1 is the central region's length in the longitudinal direction and L5 is the absorbent core's length in the longitudinal direction.
{35} The disposable diaper as set forth in clause {34}, wherein the central region's length in the longitudinal direction is from 60 to 550 mm, preferably from 100 to 500 mm.
{36} The disposable diaper as set forth in clause {34} or {35}, wherein the absorbent core's length in the longitudinal direction is from 250 to 800 mm, preferably from 300 to 700 mm.

The present invention will be described in further detail below through Examples thereof. Note, however, that the scope of the present invention is not limited to the Examples.

### Example 1:

A diaper having the same basic configuration as the diaper 1 illustrated in Figs. 1 to 3 was produced. First, an absorbent member 4 was arranged between a topsheet 2 constituted by nonwoven fabric and a backsheet 3 constituted by a resin film, and this assembly was employed as an absorbent assembly 10. Then, leak-proof cuffs 6, each formed by fixing leak-proof-cuff-forming elastic members 61 in a stretched state between a leak-proof-cuff-forming sheet 62, were provided respectively to both lateral sides of the absorbent assembly 10. An outer cover 5 was joined to the non-skin-facing surface side of the absorbent assembly 10, and the respective lateral side edge portions in the front portion A and the rear portion B of the outer cover 5 were joined together, to form a pair of side seals S, S. The absorbent member 4 of the diaper obtained as above included: an absorbent core 40 including a fiber material and a water-absorbent polymer 46; and a core-wrap sheet 48 covering the surface of the absorbent core 40. In the crotch portion C, the absorbent member included a central absorbent member 4C, a pair of side absorbent members 4S, 4S, and a pair of bending-guide portions 45, 45 each being formed between the central absorbent member 4C and the respective side absorbent member 4S. The absorbent core 40 had a central region 41 in the central absorbent member 4C, and side regions 43 in the respective side absorbent members 4S, and the central region 41 and the side regions 43 were divided by the pair of bending-guide portions 45, 45. Each bending-guide portion 45 was constituted by a slit penetrating the absorbent core 40 in the thickness direction Z. In the absorbent member 4 of the present Example, the core-wrap sheet 48 located on the absorbent core 40's skin-facing surface side and the core-wrap sheet located on the absorbent core's non-skin-facing surface side were joined together in the slit.

Table 1 below shows various specification data of the absorbent member 4 and the absorbent core 40 both before absorbing liquid and in the reference swollen state for each of the Examples 1 and 2 and Comparative Examples 1 and 2.

### Example 2:

A diaper having a configuration similar to Example 1 was produced, except that each bending-guide portion 45 was constituted by a groove having a thin portion 47. The thin portion 47's basis weight was 110 g/m².

### Comparative Example 1:

A diaper having a configuration similar to Example 1 was produced, except that an absorbent member having another bending-guide portion, in addition to the pair of bending-guide portions 45, 45, was used, and the width of the central region 41 and the width of each side region 43, 43, before absorbing liquid, were changed. The other bending-guide portion in the present Comparative Example was constituted by a slit extending along the longitudinal direction X and located between the pair of bending-guide portions 45, 45 in the width direction Y.

### Comparative Example 2:

A diaper having a configuration similar to Example 1 was produced, except that the respective widths of the central region 41, the side region 43, 43 and the bending-guide portion 45 in the absorbent core 40 before absorbing liquid were changed. Since these widths were changed, in Comparative Example 2, the entire width of the absorbent core 40 was also different from that of Example 1. In Comparative Example 2, the respective lateral side edges of each bending-guide portion had a projecting/depressed shape, and the width of each bending-guide portion was varied along the longitudinal direction. Table 1 below shows the mean value of the width.

For each of the diapers of the respective Examples and Comparative Examples, the test piece's 30-mm compression load was measured according to the aforementioned method. Also, for the side absorbent member 4S of each of the diapers of the respective Examples and Comparative Examples, the 10-mm compression load was measured according to the aforementioned method. The measurement results are also shown in Table 1 below.

**[Table 1]**

| | | | Example 1 | Example 2 | Comparative Example 1 | Comparative Example 2 |
|---|---|---|---|---|---|---|
| Before absorbing liquid | Absorbent core in crotch portion C | Absorbent core's width (mm) | 135 | 135 | 135 | 110 |
| | | Central region's width (mm) | 20 | 20 | 55 | 30 |
| | | Side region's width (mm) | 42.5 | 42.5 | 25 | 30 |
| | | Embodiment of pair of bending-guide portions | Slit | Groove | Slit | Slit |
| | | Width of pair of bending-guide portions (mm) | 15 | 15 | 15 | 8 |
| | | Number of other bending-guide portions | - | - | 1 | - |
| | | Embodiment of other bending-stride portion | . | - | Slit | - |
| | | Width of other bending-guide portion (mm) | - | - | 10 | - |
| Reference swollen state | Absorbent core in crotch portion C | Central region's width (mm) | 27 | 27 | 55 | 30 |
| | | Side region's width (mm) | 42 | 42 | 26 | 34 |
| | | Wa: Half the central region' s width (mm) | 13 | 13 | 28 | 15 |
| | | t3: Side region's thickness (mm) | 20 | 19 | 16 | 18 |
| | | Wa/t3 | 0.67 | 0.69 | 1.53 | 0.82 |
| | Diaper | Test piece's 30-mm compression load (N) | 5.77 | 6.88 | 7.52 | 10.21 |
| | Side absorbent member | 10-mm compression load (N) | 3.77 | 0.89 | 0.46 | 6.17 |

In the diapers of Examples 1 and 2, the width of the bending-guide portion 45 before absorbing liquid was 10 mm or greater; further, in the reference swollen state, the width of the side region 43 was greater than the width of the central region 41, and also, half the width of the central region 41 (indicated as "Wa" in Table 1) was 0.7 times or less of the thickness t3 of each side region 43. Further, in the diapers of Examples 1 and 2, the test piece's 30-mm compression load was less than 7 N, and the 10-mm compression load of the side absorbent member 4S was less than 4 N.

### Evaluation I of Gait Influence Degree:

Using the diapers of Example 1 and Comparative Examples 1 and 2, the gait influence degree was evaluated according to the following method including steps (A), (B), and (C). In the present evaluation method, 26 toddlers aged 18 to 20 months served as subjects.

First, in step (A), each subject was made to walk for at least three walking cycles in a state where he/she was not wearing the diaper (this state is also referred to as "non-wearing state"), and this walking state was monitored using a three-dimensional motion capture device (Vicon camera system; × 16; 200 Hz). During this monitoring, markers were attached to a plurality of sites on the subject's body, and the subject's gait was monitored by obtaining the positional information and movement information of the markers in the walking state. In this monitoring, the subject was made to walk on a ground reaction force meter (force plate; AMTI; 2000 Hz) for measuring force received from the floor. The monitoring results obtained in present step (A) were employed as results during normal gait.

Next, in step (B), the diapers of Example 1 and Comparative Examples 1 and 2 were brought into a swollen state by 160 g of physiological saline, and each subject was made to wear the swollen-state diaper. In this state, each subject was made to walk for at least three walking cycles, and this walking state was monitored using the aforementioned three-dimensional motion capture device. Also during this monitoring, the subject was made to walk on the aforementioned ground reaction force meter.

In the present step (B), physiological saline was used instead of artificial urine from the viewpoint of irritancy to the skin. More specifically, the diaper was brought into a swollen state (also referred to as "saline-swollen state") by injecting 160 g of physiological saline according to the following method. First, a mark (also referred to as "first mark") was made at the center position of an unused diaper 1 with an oil-based pen, the center position being at the center in the longitudinal direction X (i.e., the fold in the product's crotch portion) and at the center in the width direction Y. Then, another mark was made at a position 7 cm away toward the front portion A side in the longitudinal direction X from the center position marked with the first mark, and the position marked with this other mark was employed as the injection point for injecting physiological saline. Next, a section forming the diaper 1's waist opening WH was gripped, and, with the diaper 1's crotch portion C hanging down such that the center position with the first mark was located at the lowest position, 160 g of physiological saline was injected to the injection point at an injection rate of 5 g/second. A tube pump was used for this injection. Then, after injection of the physiological saline, the diaper 1 was left to stand in the hanging-down state for 1 minute, to bring the diaper into a saline-swollen state.

It should be noted that, in the present step (B), it was verified in advance that there was no difference in the diaper's dimensions and compression characteristics between the reference swollen state achieved by injecting artificial urine and the saline-swollen state achieved by injecting physiological saline.

The monitoring results obtained in present step (B) were employed as results during diaper-wearing gait.

In step (C), the results during normal gait obtained in step (A) and the results during diaper-wearing gait obtained in step (B) were compared. For this comparison, a three-dimensional image analysis software (Visual 3D C-motion) was used. More specifically, the respective measurement values of the frontal plane hip joint angle, the pelvis angle, and the amount of movement of the body's barycenter were compared between the normal gait and the diaper-wearing gait.

The "frontal plane hip joint angle" serves as an index that primarily indicates the amount of movement of the femoral joint toward the lateral side of the pelvis. Imagine a pelvic coordinate system of the pelvis having X, Y and Z axes and a femoral coordinate system of the femoral joint also having X, Y and Z axes; the "frontal plane hip joint angle" is measured, for example, as the tilt angle of the X axis or Z axis of the femoral coordinate system, which rotates about the Y axis of the pelvic coordinate system, with respect to the Y-Z plane or X-Y plane of the pelvic coordinate system. In the present evaluation method, to compare the values of the frontal plane hip joint angle-which is measured at a position where the walking distance is 80% of a single walking cycle-between the normal gait and the diaper-wearing gait, the value for the diaper-wearing gait was subtracted from the value for the normal gait. Herein, "single walking cycle" refers to a cycle in walking, from when the subject's left heel contacts the floor surface to when the left heel contacts the floor surface the next time.

The pelvis angle was found by creating, from the monitoring results, a chart showing the change in pelvis angle during a single walking cycle, and calculating the integral of the pelvis angle over the entire single walking cycle. In the present evaluation method, the integral for the diaper-wearing gait was subtracted from the integral for the normal gait, to thereby compare the normal gait and the diaper-wearing gait.

The amount of movement of the body's barycenter is the amount of movement by which the barycenter of the subject's body fluctuates in the left-right direction and the vertical direction during walking. In the present evaluation method, to compare, between the normal gait and the diaper-wearing gait, the total distance of movement of the body's barycenter in each of the left-right direction and the vertical direction during a single walking cycle, the total movement distance for the diaper-wearing gait was subtracted from the total movement distance for the normal gait.

In step (C), the calculated differences between the normal gait and the diaper-wearing gait were found for each of the subjects, and the mean value of these differences was calculated. The measurement results are shown in Table 2 below.

**[Table 2]**

| | | Example 1 | Comp arative Example 1 | Comp arative Example 2 |
|---|---|---|---|---|
| Frontal plane hip joint angle | | 1.683 | 1.624 | 4.315 |
| Pelvis angle | | -348.476 | -492.624 | -652.261 |
| Amount of movement of body's bary center | Left-right direction | -0.001 | -0.007 | -0.013 |
| | Vertical direction | 0.01 | 0.014 | 0.018 |

The results of Table 2 show that, regarding the frontal plane hip joint angle, the pelvis angle and the amount of movement of the body's barycenter, the difference between the normal gait and the diaper-wearing gait for the diaper of Example 1 was smaller than that for Comparative Examples 1 and 2. These results show that, when the diaper (absorbent core) has swollen due to urination, it is easier to walk in the diaper of Example 1 than in the diapers of Comparative Examples 1 and 2. Further, as shown in Table 1 above, in the diaper of Example 1, the test piece's 30-mm compression load was less than 7 N. With the diaper having this configuration, the integral of the pelvis angle over a single walking cycle was keep small, as shown in Table 2. This is thought to be because, in cases where the 30-mm compression load is less than 7 N, the movement around the wearer's femoral regions is less likely to be restricted. That is, the results shown in Tables 1 and 2 indicate that it is easier to walk in the diaper of Example 1, because pressure from the diaper is less likely to be applied to the insides of both legs in the wearer's crotch section, even when the absorbent core has swollen due to urination.

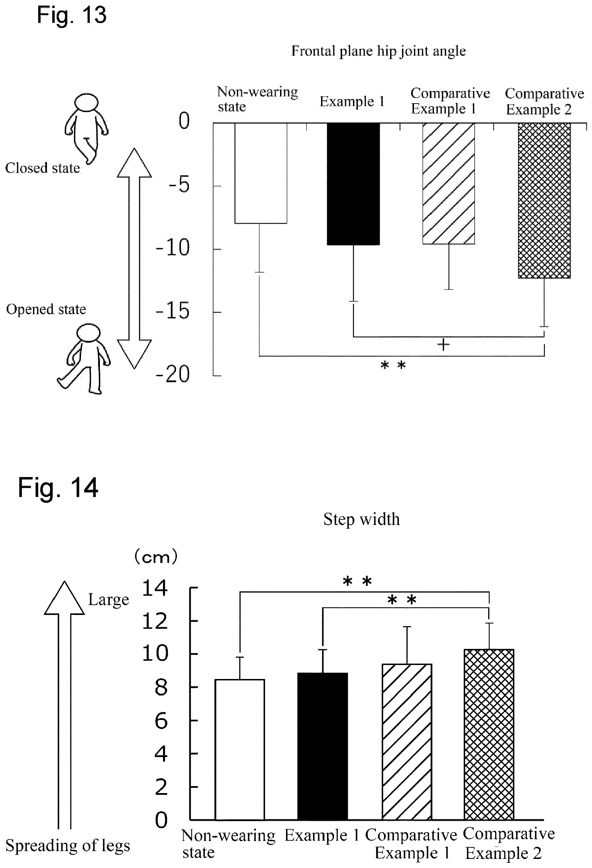

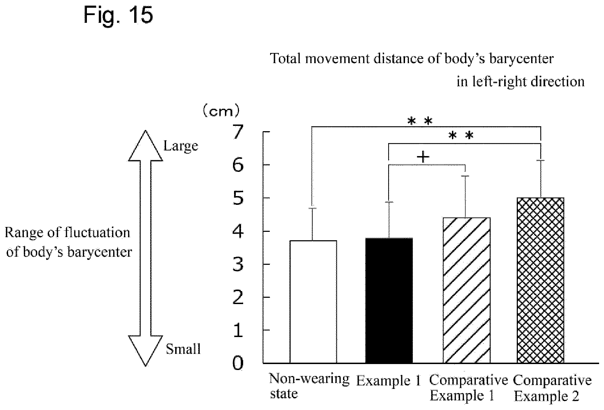

From the results of the normal gait and the diaper-wearing gait of each subject as employed in the aforementioned step (C), the mean value of the frontal plane hip joint angle and the mean value of the amount of movement of the body's barycenter (left-right direction) were calculated. The mean value of the frontal plane hip joint angle is shown in Fig. 13, and the mean value of the amount of movement of the body's barycenter (left-right direction) is shown in Fig. 15. Further, in the aforementioned steps (A) and (B), in addition to monitoring walking of each subject, the subject's step width was measured using the aforementioned ground reaction force meter. The "step width" is the distance along the orthogonal direction-which is defined as the direction orthogonal to the forward movement direction during walking-between a straight line connecting the ground-contact positions of the heel of one foot which are lined up along the forward movement direction during walking and a straight line connecting the ground-contact positions of the heel of the other foot which are lined up along the forward movement direction during walking. From the measurement values of the step width of the respective subjects, the mean value of the step width was calculated. The mean value of the step width is shown in Fig. 14. The frontal plane hip joint angle, the step width, and the total movement distance of the body's barycenter in the left-right direction were statistically analyzed by one-way analysis of variance. Also, the level of significance was adjusted by the Bonferroni method with consideration given to multiplicity.

The frontal plane hip joint angle shown in Fig. 13 indicates that, the closer the value is to 0, the more the hip joint is closed, whereas the farther the value is from 0, the more the hip joint is opened.

The step width shown in Fig. 14 indicates that, the greater the value, the more the left and right legs are spread apart during walking.

The movement distance of the body's barycenter in the left-right direction shown in Fig. 15 indicates that, the greater the distance, the greater the range of fluctuation of the body's barycenter in the left-right direction.

In Figs. 13 to 15, the mark "**" (two asterisks) indicates that a significant difference was found between the two test examples being compared at a level of significance of p<0.01/N; the mark "*" (one asterisk) indicates that a significant difference was found at a level of significance of p<0.05/N; and the mark "+" (one plus sign) indicates that no significant difference was found but the level was at p<0.1/N. (N is the number of times of tests.)

As illustrated in Fig. 13, the result regarding the frontal plane hip joint angle for the diaper of Example 1 in the saline-swollen state was significantly closer to 0 compared to the diaper of Comparative Example 2 in the saline-swollen state. Further, as illustrated in Fig. 14, the result regarding the step width for the diaper of Example 1 in the saline-swollen state was significantly narrower compared to the diaper of Comparative Example 2 in the saline-swollen state. The results of Figs. 13 and 14 show that, compared to the diaper of Comparative Example 2 in the saline-swollen state, the diaper of Example 1 in the saline-swollen state suppresses the hip joint from opening and both legs from spreading open during walking, and the opening of the hip joint and the spreading between both legs are closer to the values for the aforementioned non-wearing state.

As illustrated in Fig. 15, the result was that the total movement distance of the body's barycenter in the left-right direction was significantly shorter and the range of fluctuation of the body's barycenter was smaller for the diaper of Example 1 in the saline-swollen state compared to the diapers of Comparative Examples 1 and 2 in the saline-swollen state. The results show that the total movement distance of the body's barycenter in the left-right direction for the diaper of Example 1 in the saline-swollen state is closer to the value for the non-wearing state.

From the results of Figs. 13 to 15, it can be assessed that the diaper of Example 1 enables walking in a manner similar to the non-wearing state and is thus easy to walk in, even when the absorbent core 40 has swollen due to urination.

### Evaluation II of Gait Influence Degree:

In the present evaluation method, 14 toddlers aged 18 to 20 months served as subjects. First, each subject was made to walk in the non-wearing state, and also in states of wearing the respective diapers of Example 2 and Comparative Examples 1 and 2 in the saline-swollen state, and respective motion videos of the subject's walk were captured. At this time, the subject was made to walk on the aforementioned ground reaction force meter. Also in this evaluation method, the saline-swollen state was created by injecting physiological saline to each diaper according to the same method as in the aforementioned Evaluation I of gait influence degree.

Next, OpenPose (URL=https://gthub.com/CMU-Perceptual-Computing-Lab/poenpose) was used to obtain skeleton information from the motion video data of each subject's walk. The skeleton information includes, for example: composite data created by extracting various parts within the motion video data, such as the neck, shoulders, elbows, wrists, hip, knees, ankles, etc., and rendering those extracted parts as points and lines by synchronizing them with the motion video data; and three-dimensional coordinate information on those parts. Based on the skeleton information, the step width (cm) and knee-to-knee distance (cm) of each of the subjects during walking were calculated, and the mean value thereof was calculated. The step width based on the skeleton information was found by measuring, between the left and right femurs, the distance between the midpoint of a line connecting the outermost protrusion of the lateral femoral epicondyle and the innermost protrusion of the medial femoral epicondyle for the left femur and the same midpoint for the right femur. In this evaluation method, in addition to measuring the step width (cm) based on the skeleton information, the step width of each of the subjects was measured using the ground reaction force meter as in the Evaluation I of gait influence degree, and the mean value thereof was calculated.

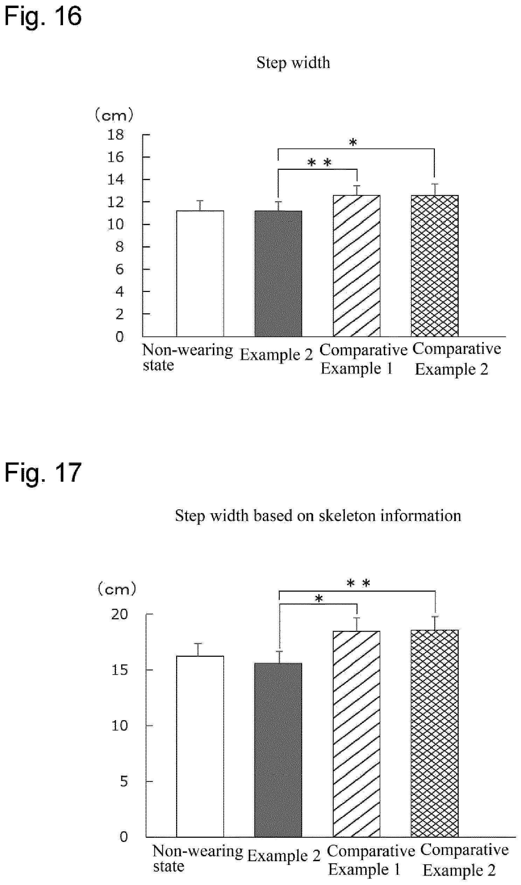

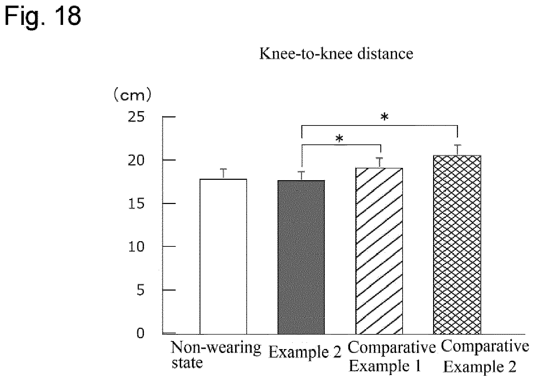

Fig. 16 shows the step width measured with the ground reaction force meter. Fig. 17 shows the step width based on the skeleton information during walking. Fig. 18 shows the knee-to-knee distance. As for the step width and the knee-to-knee distance, the statistical difference (significant difference) between two test examples was verified by the paired t-test.

The knee-to-knee distance shown in Fig. 18 indicates that, the greater the value, the more the left and right knees are spread apart during walking.

In Figs. 16 to Fig. 18, the mark "**" (two asterisks) indicates that a significant difference was found between the two test examples being compared at a level of significance of p<0.01; and the mark "^{∗}" (one asterisk) indicates that a significant difference was found at a level of significance of p<0.05.

The results of Figs. 16 and 17 show that the diaper of Example 2 in the saline-swollen state had a significantly narrower step width compared to the diapers of Comparative Examples 1 and 2 in the saline-swollen state. The results of Figs. 16 and 17 show that, compared to the diapers of Comparative Examples 1 and 2 in the saline-swollen state, the diaper of Example 2 in the saline-swollen state suppresses both legs from spreading open, and the spreading between both legs is closer to the value for the aforementioned non-wearing state. Further, since the step width found based on the skeleton information as shown in Fig. 17 had similar results as the step width found using a ground reaction force meter as shown in Fig. 16, it was verified that the gait influence degree could be evaluated also according to the step width found based on the skeleton information.

The results of Fig. 18 show that the diaper of Example 2 in the saline-swollen state had a significantly narrower knee-to-knee distance compared to the diapers of Comparative Examples 1 and 2 in the saline-swollen state. Further, the results also show that, in the diaper of Example 2 in the saline-swollen state, the knee-to-knee distance is close to the value for the aforementioned non-wearing state.

From the results of Figs. 16 to 18, it can be assessed that the diaper of Example 2 enables walking in a manner similar to the non-wearing state and is thus easy to walk in, even when the absorbent core 40 has swollen due to urination.

### Industrial Applicability

With the disposable diaper of the present invention, the wearer's movement around the femoral region is less likely to be restricted, even when the absorbent core has absorbed liquid and has swollen, thereby making it easy to walk in.

## Claims

1. A disposable diaper comprising a topsheet, a backsheet, and an absorbent member arranged between the topsheet and the backsheet,
the absorbent member including an absorbent core,
the disposable diaper having a longitudinal direction and a width direction orthogonal to the longitudinal direction,
the disposable diaper having a rear portion to be arranged on a wearer's rear side when worn, a front portion to be arranged on the wearer's front side when worn, and a crotch portion located between the front portion and the rear portion, wherein:
in the crotch portion, the absorbent member includes
a central absorbent member extending in the longitudinal direction,
a pair of side absorbent members respectively located on both sides, in the width direction, of the central absorbent member, the side absorbent members being configured to stand up from a skin-facing surface side of the central absorbent member in a worn state, and
a pair of bending-guide portions, each of the bending-guide portions being formed between the central absorbent member and the respective side absorbent member;
the absorbent core has a central region in the central absorbent member and side regions in the respective side absorbent members, the central region and the side regions being divided by the pair of bending-guide portions;
the bending-guide portion is constituted by a slit or a groove;
a width of the bending-guide portion before absorbing liquid is from 10 to 30 mm;
in a reference swollen state achieved by injecting 160 g of artificial urine to an injection point located 7 cm away toward the front portion side in the longitudinal direction from a center position of the disposable diaper, which is at a center in the longitudinal direction and at a center in the width direction, and leaving the disposable diaper to stand for 5 minutes, a width of the side region of the absorbent core is greater than a width of the central region; and
in the reference swollen state, half the width of the central region is 0.7 times or less of a thickness of each said side region.

2. The disposable diaper according to claim 1, wherein, in a test piece obtained by folding the disposable diaper in the reference swollen state along the longitudinal direction in a manner that the disposable diaper's width is divided equally in two, compression load to compress the test piece until the test piece's thickness in the crotch portion becomes 30 mm is 7 N or less.

3. The disposable diaper according to claim 1 or 2, wherein, in the reference swollen state, compression load to compress at least one of the pair of side absorbent members until the thickness thereof becomes 10 mm is 4.5 N or less.

4. The disposable diaper according to any one of claims 1 to 3, wherein, when the absorbent member is folded in the reference swollen state along the longitudinal direction in a manner that the absorbent member's width is divided equally in two, the absorbent member's thickness in the crotch portion is 60 mm or less.

5. The disposable diaper according to any one of claims 1 to 4, wherein the absorbent core has a two-layer structure.

6. The disposable diaper according to any one of claims 1 to 5, wherein the side region's basis weight is equal to or greater than the central region's basis weight.

7. The disposable diaper according to any one of claims 1 to 6, wherein a ratio B2B 1 of B2 to B1 is from 1 to 3, where B1 is the central region's basis weight and B2 is the side region's basis weight.

8. The disposable diaper according to any one of claims 1 to 7, wherein the central region's basis weight is from 80 to 650 g/m².

9. The disposable diaper according to any one of claims 1 to 8, wherein the side region's basis weight is from 80 to 700 g/m².

10. The disposable diaper according to any one of claims 1 to 9, wherein, in the reference swollen state, a ratio W2/W1 of W2 to W1 is from 1.1 to 5, where W1 is the central region's width and W2 is the side region's width.

11. The disposable diaper according to any one of claims 1 to 10, wherein, in the reference swollen state, the central region's width is from 8 to 35 mm.

12. The disposable diaper according to any one of claims 1 to 11, wherein, in the reference swollen state, the side region's width is from 35 to 60 mm.

13. The disposable diaper according to any one of claims 1 to 12, wherein the width of the bending-guide portion before absorbing liquid is from 13 to 20 mm.

14. The disposable diaper according to any one of claims 1 to 13, wherein, in the reference swollen state, the central region's thickness is from 5 to 30 mm.

15. The disposable diaper according to any one of claims 1 to 14, wherein, in the reference swollen state, the side region's thickness is from 10 to 30 mm.

16. The disposable diaper according to any one of claims 1 to 15, wherein the absorbent member includes a bending-guide portion other than the pair of bending-guide portions.

17. The disposable diaper according to any one of claims 1 to 16, wherein the absorbent core has a narrowed portion where both lateral side edges of the absorbent core extending along the longitudinal direction are narrowed inwardly in the width direction, the narrowed portion being located more toward the front portion side than the center in the longitudinal direction.

18. The disposable diaper according to any one of claims 1 to 17, wherein:
the width of each said bending-guide portion varies along the longitudinal direction; and
each said bending-guide portion has a maximum width portion where the width becomes the greatest, the maximum width portion being located more toward the front portion side than the center in the longitudinal direction.

19. The disposable diaper according to any one of claims 1 to 18, wherein the bending-guide portion is formed rectilinearly.

20. The disposable diaper according to any one of claims 1 to 18, wherein the bending-guide portion is formed curvilinearly.

21. The disposable diaper according to any one of claims 1 to 20, further comprising an absorbent assembly including the topsheet and the absorbent member, and further including a pair of leak-proof cuffs on respective lateral sides extending along the longitudinal direction,
wherein, in the width direction, a length of a section where the leak-proof cuff overlaps the absorbent core is shorter than the side region's width before absorbing liquid.

22. The disposable diaper according to any one of claims 1 to 21, wherein:
the absorbent member includes a core-wrap sheet covering the absorbent core;
the bending-guide portion is constituted by a slit; and
the core-wrap sheet located on the absorbent core's skin-facing surface side and the core-wrap sheet located on the absorbent core's non-skin-facing surface side are joined together in the slit.

23. The disposable diaper according to claim 22, wherein the core-wrap sheet on the skin-facing surface side and the core-wrap sheet on the non-skin-facing surface side are joined together also in the reference swollen state.

24. The disposable diaper according to claim 22 or 23, wherein a joining strength between the core-wrap sheet is 5 cN/10 mm or greater.

25. The disposable diaper according to any one of claims 1 to 21, wherein:
the bending-guide portion is constituted by a groove;
the absorbent core has a thin portion located on the groove's non-skin-facing surface side and between the central region and each said side region; and
the thin portion's basis weight is from 1 to 120 g/m².

26. The disposable diaper according to claim 25, wherein, in the reference swollen state, a ratio t3/t4 of t3 to t4 is 2 or greater, where t3 is the side region's thickness and t4 is the thin portion's thickness.
